# EUROPEAN PATENT SPECIFICATION

(11) **EP 4 281 583 B1**
(45) Date of publication and mention of the grant of the patent: **20.05.2026**
(21) Application number: 22742960.2
(22) Date of filing: 20.01.2022
(51) Int. Cl.: C12Q 1/6806

(54) **HEATRICH-BS: HEAT ENRICHMENT OF CPG-RICH REGIONS FOR BISULFITE SEQUENCING**
HEAT-RICH-BS: WÄRMEANREICHERUNG VON CPG-REICHEN REGIONEN ZUR BISULFITSEQUENZIERUNG
HEATRICH-BS : ENRICHISSEMENT THERMIQUE DE RÉGIONS RICHES EN CPG POUR SÉQUENÇAGE AU BISULFITE

(30) Priority: 20.01.2021 SG 10202100612X
(43) Date of publication of application: 29.11.2023
(73) Proprietor: National University of Singapore, Singapore 119077 (SG); Singapore Health Services Pte Ltd, Singapore 168582 (SG)
(72) Inventor: CHEOW, Lih Feng, Singapore 119077 (SG); CHERUBA, Elsie, Singapore 119077 (SG); TAN, Bee Huat Iain, Singapore 169610 (SG); CHONG, Qingqing Dawn, Singapore 169610 (SG)
(74) Representative: Marks & Clerk LLP
(86) International application number: PCT/SG2022/050021
(87) International publication number: WO 2022/159035

(56) References cited:
- WO-A1-2007/018602
- HONGCANG GU ET AL: "Preparation of reduced representation bisulfite sequencing libraries for genome-scale DNA methylation profiling", NATURE PROTOCOLS, vol. 6, no. 4, 1 March 2011 (2011-03-01), pages 468 - 481, XP055151116, ISSN: 1754-2189, DOI: 10.1038/nprot.2010.190
- SHULI KANG ET AL: "CancerLocator: non-invasive cancer diagnosis and tissue-of-origin prediction using methylation profiles of cell-free DNA", GENOME BIOLOGY, vol. 18, no. 1, 24 March 2017 (2017-03-24), XP055682390, DOI: 10.1186/s13059-017-1191-5
- DE KOKER A. ET AL.: "A versatile method for circulating cell -free DNA methylome profiling by reduced representation bisulfite sequencing", BIORXIV, 25 February 2022 (2022-02-25), pages 1 - 26, XP055830009, DOI: 10.1101/663195
- WENYUAN LI, QINGJIAO LI, SHULI KANG, MARY SAME, YONGGANG ZHOU, CAROL SUN, CHUN-CHI LIU, LEA MATSUOKA, LINDA SHER, WING HUNG WONG, : "CancerDetector: ultrasensitive and non-invasive cancer detection at the resolution of individual reads using cell-free DNA methylation sequencing data", NUCLEIC ACIDS RESEARCH, OXFORD UNIVERSITY PRESS, GB, vol. 46, no. 15, 6 September 2018 (2018-09-06), GB , pages e89 - e89, XP055692134, ISSN: 0305-1048, DOI: 10.1093/nar/gky423
- LIU M.C. ET AL.: "Sensitive and specific multi-cancer detection and localization using methylation signatures in cell -free DNA", ANN ONCOL, vol. 31, no. 6, 30 March 2020 (2020-03-30) - 25 February 2022 (2022-02-25), pages 745 - 759, XP055809431, DOI: 10.1016/J.ANNONC. 2020.02.01 1
- CHERUBA E. ET AL.: "Heatrich-BS enables efficient CpG enrichment and highly scalable cell -free DNA methylation profiling", BIORXIV, 6 April 2021 (2021-04-06) - 25 February 2022 (2022-02-25), pages 1 - 19, XP055957897, DOI: 10.1101/ 2021.04.05.437976

## Description

### FIELD OF THE INVENTION

The present invention is directed to a method using heat denaturation of DNA fragments having low GC content to enrich for CpG regions that can be enriched by adapter-ligation, subjected to bisulfite conversion, sequenced and analysed, for example, to detect cancer.

### BACKGROUND OF THE INVENTION

Liquid biopsy has tremendous value in the screening and monitoring of cancer. The full potential of liquid biopsy can only be realized with highly sensitive, specific and cost-effective methods that can identify the small fractions of tumor DNA in the blood. The most widely used method to identify circulating tumor DNA (ctDNA) relies on the detection of tumor-derived mutations in cell-free DNA [Forshew, T., et al. Sci. Transl. Med. 4 (2012)]. The key challenge associated with this approach is that mutation-bearing tumor fragments constitute less than 0.01% of cell-free DNA [Fiala, C. and Diamandis, E.P. BMC Med., 16, 1-10 (2018)]. In order to detect these rare fragments, digital PCR approaches [Diehl, F., et al., Nat. Methods, 3, 551-559 (2006)] or extremely deep sequencing (>300x) [Shu, Y., et al. Sci. Rep., 7, 1-11 (2017)] have been successfully used. The downside of these approaches is that they are impossible to perform on a genome-wide scale, making the assays targeted in nature. As a result, novel or unknown mutations cannot be detected. Furthermore, the pervasiveness of even the most well-known cancer-specific mutations is low between patients, limiting the value of targeted assays and the sensitivity of tumor detection [Bos, J.L., et al., Nature, 327, 293-297 (1987)]. For example, despite being one of the most common oncogenes, only 40% of colorectal cancer patients carry the RAS mutation [Bos, J.L., et al., Nature, 327, 293-297 (1987)]. Therefore, while targeted deep sequencing can be a sensitive method to monitor tumors with known mutations, it can only detect a fraction of the positive patients when used for screening. Furthermore, targeted sequencing methods are also limited by the panel of known mutations. On the other hand, genome-wide sequencing can detect all the carried mutations, eliminating the need for prior knowledge of the targets. But performing genome-wide sequencing at the required depth is not feasible due to the high cost involved. To overcome these issues, ctDNA detection assays based on other properties such as fragment size [Mouliere, F., et al. Sci. Transl. Med., 10, 1-14 (2018)], fragmentation pattern [Cristiano, S., et al. Nature, 570, 385-389 (2019)] and methylation patterns have been developed [Guo, S., et al., Nat. Genet., 49, 635-642 (2017)], some of which have been commercialized.

Given the limitations of mutation-based ctDNA assays, and the role of methylation in cancer development and progression, methylation-based ctDNA assays are emerging as a possible alternative. The utility of methylation markers to detect ctDNA in blood has been well established, with one such assay to detect hypermethylated septin9 for colorectal cancer being FDA-approved. However, this assay has a sensitivity of only 70% and false positive rate of 20% [Johnson, D. A. et al. PLoS One 9, e98238 (2014)] as studies have shown that methylated septin 9 is only present in about 70% of colorectal cancer patients, and septin 9 methylation is not specific to colorectal cancer. Large scale programs are also being established to develop and validate methylation-based ctDNA assays for cancer screening. One such program is the Circulating Cell-free Genome Atlas (CCGA), which has successfully shown that targeted methylation assays, in combination with machine learning algorithms, can be used to detect ctDNA fragments and localize to the tissue of origin [Liu, M.C., et al. Ann. Oncol., 31, 745-759 (2020)]. This study has also shown that methylation-based detection has outperformed genome-wide and targeted mutation assays in cancer detection and tissue of origin localization. Such improved detection can be achieved because methylation-based ctDNA assays offer the following advantages: (i) Methylation patterns are ubiquitous, and can be specific to the tissue and type of cancer. This enables the use of published sequencing data from resected tumors to generate a unique methylation signature for the cancer of interest [Moss, J., et al. Nat. Commun., 9 (2018)]. (ii) The methylome of cancer cells exhibit a distinct pattern of methylation changes - hypermethylation of CGls and hypomethylation of the genome [Sproul, D. and Meehan, R.R. Brief. Funct. Genomics, 12, 174-190 (2013)]. (iii) Enrichment of informative regions based on methylation status, such as selection of methylated CGIs, can allow for sensitive detection of small tumor fractions [Guo, S., et al., Nat. Genet., 49, 635-642 (2017)]. The downside of current enrichment methods is that they are either targeted in nature, limiting the sites that can be assayed, or heavily dependent on methylation status, such as Methylated DNA Immunoprecipitation (MeDIP). The traditional approach to enrich for CGls is Reduced Representation Bisulfite Sequencing (RRBS), which uses methylation-insensitive *Mspl* to cleave DNA at CCGG sites, generating fragments with CpG sites at both ends [Gu, H., et al., Nat. Protoc., 6, 468-481 (2011)]. Single-cell RRBS (scRRBS) is a modification of the RRBS protocol, where all the steps are combined into a single-tube reaction to minimize loss [Guo, H., et al., Nat. Protoc., 10, 645-59 (2015)]. The scRRBS protocol has been used for cell-free DNA methylation profiling [Guo, S., et al., Nat. Genet., 49, 635-642 (2017)]. This approach is still limited in utility when used on fragmented DNA, as very few fragments can meet the requirement for a *Mspl* cut site on both ends. For example, one study that uses scRRBS to assay for methylation in cell-free DNA, henceforth referred to as cell-free RRBS, has only 6.4% of the reads in CGls [Guo, S., et al., Nat. Genet., 49, 635-642 (2017)].

Kang et al: (2017: Genome Biology) relates to non-invasive cancer diagnosis and tissue-of-origin prediction using methylation profiles of cell-free DNA.

There is a need for improved methods for increasing sensitivity and reducing cost of using circulating DNA for cancer detection and screening assays for liquid biopsy.

### SUMMARY OF THE INVENTION

The present invention is directed to methods for enriching CpG-rich regions of circulating cell-free DNA, that are known to harbor significant methylation changes in cancer irrespective of their methylation status, to provide methylation information that can be used for cancer-specific and other screening.

According to a first aspect, the present invention provides a method of enrichment of CpG islands comprising cancer-specific methylation information in isolated circulating cell-free DNA from a subject, comprising the steps:
i) provide a cell-free DNA sample;
ii) repair double-stranded DNA ends and add dA tail;
iii) heat-denature the cell-free DNA, wherein low GC content fragments are denatured while high GC content fragments remain double-stranded;
iv) ligate methylated adapters to both ends of the double-stranded DNA;
v) perform bisulfite conversion of the adapter-ligated DNA;
vi) amplify the bisulfite-converted adapter-ligated DNA from v);
vii) Size select for 190-400 bp fragments of the amplified DNA of vi).

In some embodiments, said low GC content fragments have lower than about 60% GC content and high GC content fragments have about 60% or higher GC content.

In some embodiments, the heat denaturing in step iii) is performed at a temperature in the range of about 87-92 °C.

In some embodiments, the cell-free DNA sample is from a subject that has a medical condition.

In some embodiments, the method further comprises determining at least part of the sequence of one or more of the amplified molecules.

In some embodiments, the determining of at least part of the sequence comprises paired-end sequencing.

In some embodiments, the determining step provides diagnostic information for the subject.

In some embodiments, the diagnostic information comprises cancer diagnosis information for the subject.

According to a second aspect, the present invention provides a method to determine the tumor fraction in a circulating cell-free DNA sample from a subject, the method comprising:
A) Identify differentially methylated clusters from a comparison of Whole Genome Bisulfite Sequencing datasets for normal plasma with a reference cancer methylation dataset;
B) Compare each CpG site in each bisulfite-sequenced heat-enriched CpG-rich DNA fragment from said subject sample against the reference dataset and calculate, using a bimodal distribution, a class-specific probability for each bisulfite-sequenced heat-enriched CpG-rich DNA fragment; and
C) Approximate the tumor fraction in said subject sample using maximum likelihood estimation.

In some embodiments of the second aspect, the method comprises:
A) identifying differentially methylated clusters in circulating cell-free DNA between normal subjects and subjects with cancer by;
   (i) obtaining a normal plasma whole genome bisulfite sequencing methylation dataset;
   (ii) obtaining a reference cancer methylation dataset and extrapolating, to ± 100 bp of each of a plurality of probe sites, methylation values with a standard deviation less than 0.4 between the various samples in the dataset;
   (iii) identifying differentially methylated clusters from datasets (i) and (ii);
B) determining the class-specific probability of each site using a bimodal distribution by;
   i) assigning to each sequenced fragment from the subject sample a normal and tumor class-specific proportional methylation status using the generated reference;
   ii) for every site in the reference, the contribution from the unmethylated and methylated mode (0 and 1) is calculated, wherein the relative contributions of each mode in the two classes is used to assign normal or tumor class-specific probabilities for the methylation values in the assayed fragment; and
C) estimating the tumor fraction, denoted as *θ*, where 0 ≤ *θ* < 1, of the sample;

wherein, each read is assumed to be independent, and normal and tumor class-specific probability is assigned to each read;
a global tumor fraction (*θ_{g}*) is calculated by applying a grid search to determine the highest probability event from a range of tumor fractions.

In some embodiments, in A)(iii) all CpG sites within clusters that have 0.5 difference in methylation are selected.

In some embodiments, the comparison population is selected from a group comprising circulating cell-free DNA from normal patients and/or cancer patients.

In some embodiments, the reference cancer methylation dataset is a colorectal adenocarcinoma (COAD) dataset.

According to a third aspect, the present invention provides a method of obtaining information in relation to a medical condition of a subject, the method comprising:
i) provide an isolated cell-free DNA sample from the subject;
ii) repair double-stranded DNA ends and add dA tail;
iii) heat-denature the cell-free DNA, wherein low GC content fragments are denatured while high GC content fragments remain double-stranded;
iv) ligate methylated adapters to both ends of the double-stranded DNA;
v) perform bisulfite conversion of the adapter-ligated DNA;
vi) amplify the bisulfite-converted adapter-ligated DNA from v);
vii) Size select for 190-400 bp fragments of the amplified DNA of vi);
viii) determine at least part of the sequence of one or more of the amplified molecules;
determine the tumor fraction in the sample from the subject, comprising:
A) Identify differentially methylated clusters from a comparison of Whole Genome Bisulfite Sequencing datasets for normal plasma with a reference cancer methylation dataset;
B) Compare each CpG site in each bisulfite-sequenced heat-enriched CpG-rich DNA fragment from said subject sample against the reference dataset and calculate, using a bimodal distribution, a class-specific probability for each bisulfite-sequenced heat-enriched CpG-rich DNA fragment; and
C) Approximate the tumor fraction in said subject sample using maximum likelihood estimation.

In some embodiments, low GC content fragments have lower than about 60% GC content and high GC content fragments have about 60% or higher GC content.

In some embodiments, the method of the third aspect comprises:
A) identifying differentially methylated clusters in circulating cell-free DNA between normal subjects and subjects with cancer by;
   (i) obtaining a normal plasma whole genome bisulfite sequencing methylation dataset;
   (ii) obtaining a reference cancer methylation dataset and extrapolating, to ± 100 bp of each of a plurality of probe sites, methylation values with a standard deviation less than 0.4 between the various samples in the dataset;
   (iii) identifying differentially methylated clusters from datasets (i) and (ii);
B) determining the class-specific probability of each site using a bimodal distribution by;
   i) assigning to each sequenced fragment from the subject sample a normal and tumor class-specific proportional methylation status using the generated reference;
   ii) for every site in the reference, the contribution from the unmethylated and methylated mode (0 and 1) is calculated, wherein the relative contributions of each mode in the two classes is used to assign normal or tumor class-specific probabilities for the methylation values in the assayed fragment; and
C) estimating the tumor fraction, denoted as *θ*, where 0 ≤ *θ* < 1, of the sample;

wherein, each read is assumed to be independent, and normal and tumor class-specific probability is assigned to each read;
a global tumor fraction (*θ_{g}*) is calculated by applying a grid search to determine the highest probability event from a range of tumor fractions.

In some embodiments, in A)(iii) all CpG sites within clusters that have 0.5 difference in methylation are selected.

In some embodiments, the heat denaturing in step iii) is performed at a temperature in the range of about 87-92 °C.

In some embodiments, the determining of at least part of the sequence comprises paired-end sequencing.

In some embodiments, the determining step provides diagnostic information for the subject.

In some embodiments, the diagnostic information comprises cancer diagnosis information for the subject.

In some embodiments, the comparison population is selected from a group comprising circulating cell-free DNA from normal patients and/or cancer patients.

In some embodiments, the reference cancer methylation dataset is a colorectal adenocarcinoma (COAD) dataset.

In some embodiments, the method further comprises treating the subject based on whether or not the subject is identified as having a tumor fraction indicative of a cancer.

In some embodiments, the method is capable of >98% predictive accuracy from as low as 3 million sequencing reads.

In some embodiments, the method is capable of detecting a 0.2% tumor fraction at a probability of 0.82.

### BRIEF DESCRIPTION OF THE FIGURES

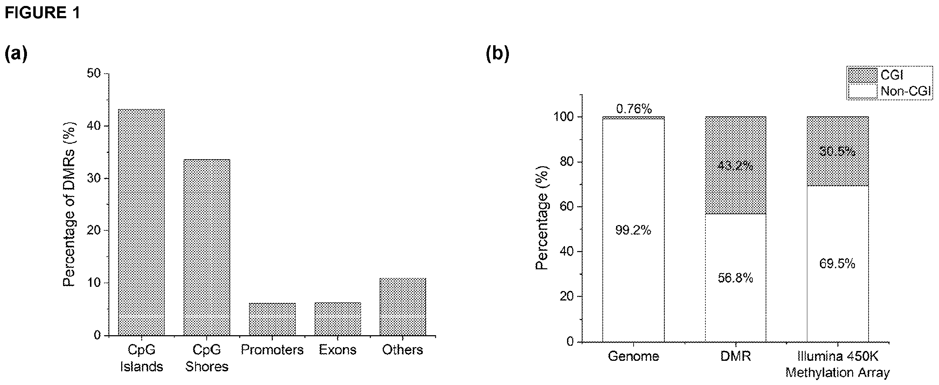

**Figure 1a****-d** shows enrichment of CpG-dense regions using the relationship with GC content. **(a)** Percentage of differentially methylated sites in different genomic regions. **(b)** Proportion of DMRs and Illumina 450k methylation array probes in CGI with respect to the genomic distribution. **(c)** Relationship between GC content and number of CpGs in randomly generated 200 bp fragments of the human genome. **(d)** Number of DMRs of different cancers detected per 1000 fragments using different GC-content thresholds. COAD: colorectal adenocarcinoma, BRCA: breast invasive carcinoma, LUAD: lung adenocarcinoma, KIRC: kidney renal clear cell carcinoma, UCEC: uterine corpus endometrial carcinoma. Fragments above 0.6 GC content contain nearly 8-fold more DMRs across different cancers.

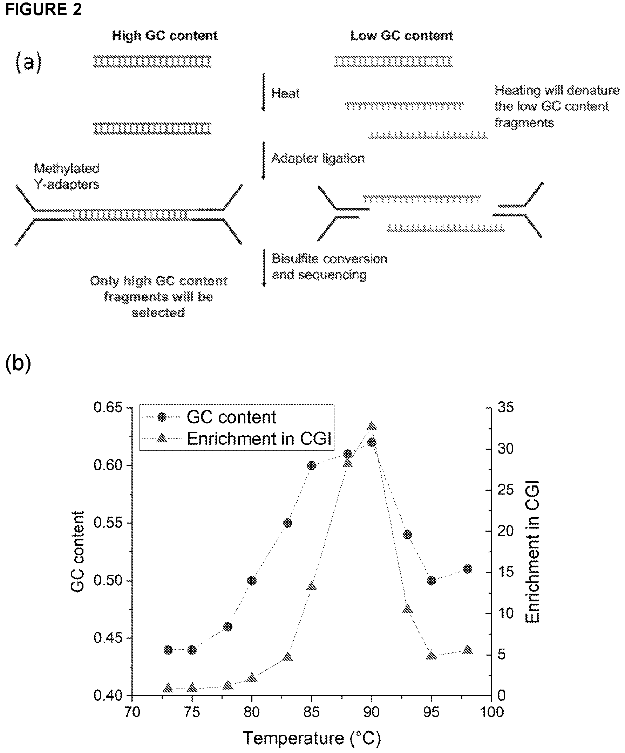

**Figure 2a****-f** shows selection of CpG-rich fragments using heat denaturation. **(a)** Workflow of Heatrich-BS to select for GC-rich fragments. **(b)** Trend of GC content (upper curve) and read enrichment at CGI (lower curve) over a range of temperatures. **(c)** Average GC content of sequenced fragments with and without heat denaturation. **(d)** Distribution of Heatrich and RRBS reads in CpG islands, shores and other regions. **(e)** Localization of Heatrich-BS and RRBS reads to CGI regions. CGls are marked with solid bars. **(f)** Distribution of RRBS reads in different genomic regions (left). Distribution of Heatrich reads in different genomic regions (right).

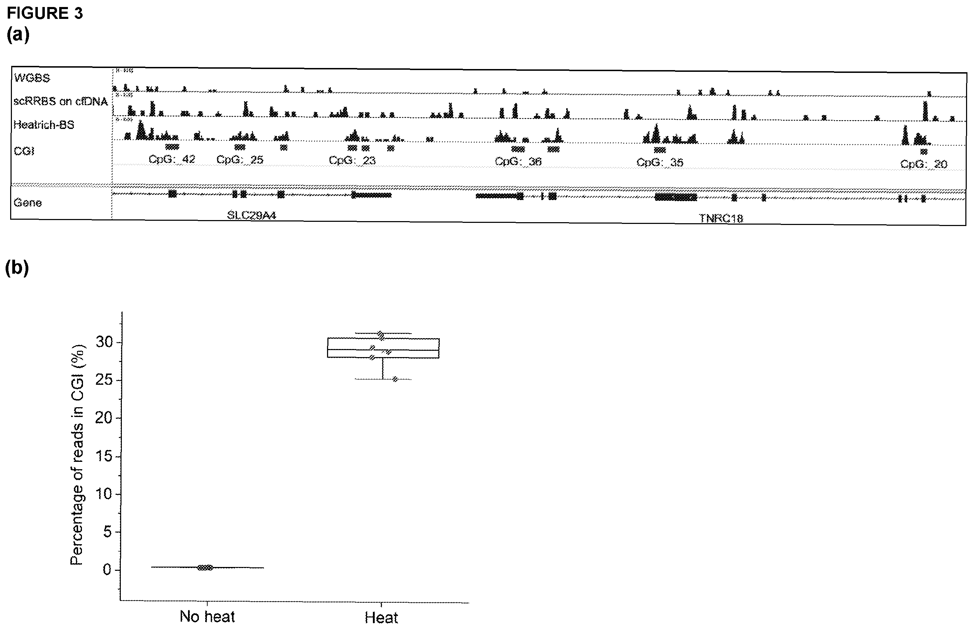

**Figure 3a****-d** shows Heatrich-BS effectively enriches for reads in CpG-rich regions. **(a)** Piling up of reads and localization to CGI using WGBS, RRBS and Heatrich-BS methods on circulating DNA. CGI regions are marked by solid bars. **(b)** Percentage of reads in CGI with and without heat denaturation. **(c)** Percentage of reads in CGI using Heatrich-BS, RRBS and WGBS on circulating DNA. **(d)** Number of reads in DMR for different total reads with and without heat denaturation.

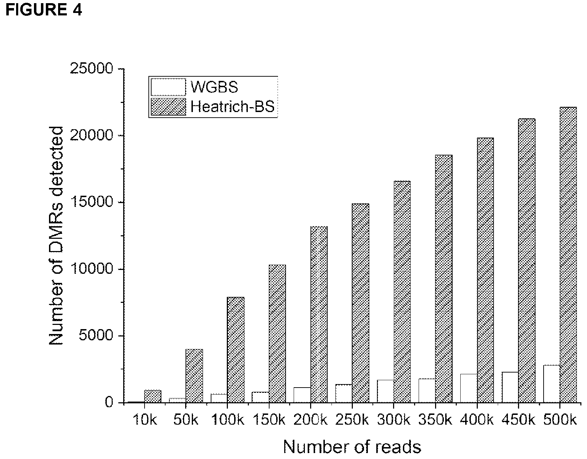

**Figure 4** shows the number of DMRs detected for different total reads with and without heat denaturation.

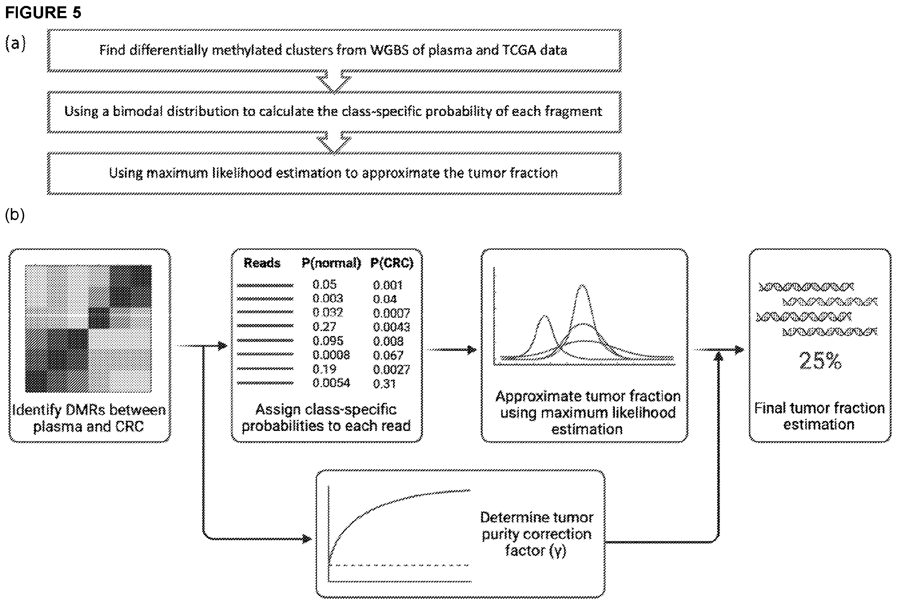

**Figure 5a****-i** shows the development and validation of a tumor fraction prediction algorithm using Heatrich-BS data. **(a)** Workflow of tumor fraction prediction algorithm. **(b)** Workflow of the tumor fraction prediction algorithm. DMRs were identified by comparing healthy volunteer plasma with TCGA CRC methylation array data. Class-specific probabilities were assigned to each sequencing fragment and maximum likelihood estimation was used to infer global tumor fraction. Tumor purity correction was applied to account for normal cell infiltration in TCGA data. **(c)** ROC analysis of 0.5% tumor fraction WGBS simulated dataset at different sequencing depths. **(d)** ROC analysis of 0.5% tumor fraction Heatrich-BS simulated dataset at different sequencing depths. **(e)** Probability of detection of different tumor fractions using WGBS simulated datasets at different sequencing depths. **(f)** Probability of detection of different tumor fractions using Heatrich-BS simulated datasets at different sequencing depths. **(g)** True and algorithm predicted values of simulated plasma WGBS cfDNA samples at different sequencing depths. Confident tumor fraction prediction is achieved beyond 150 million reads (5X sequencing depth). **(h)** True and algorithm predicted value of simulated Heatrich-BS samples using different total sequencing reads. Confident tumor fraction prediction is achieved with as few as 3 million reads. **(i)** ROC analysis of 0.5% tumor fraction using simulated Heatrich-BS and WGBS cfDNA samples at 3 million total reads. AUC of Heatrich-BS at 3 million reads much higher (0.988) compared to AUC of WGBS (0.547).

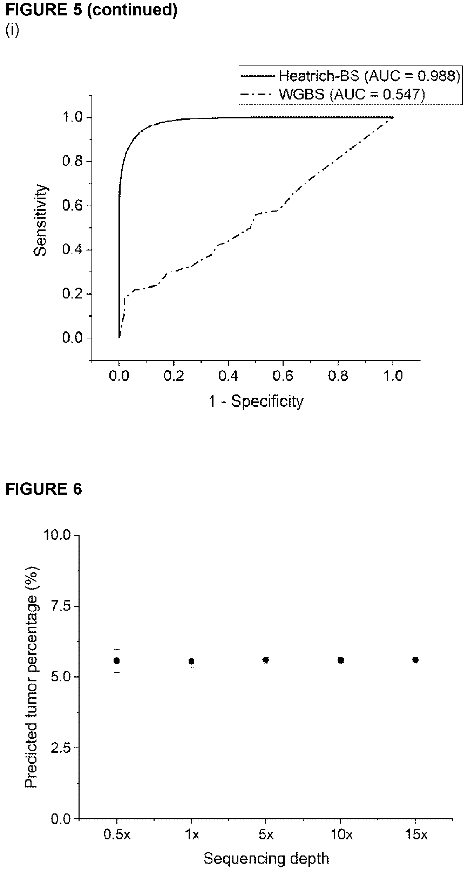

**Figure 6** shows the baseline tumor fraction of normal samples at different sequencing depths.

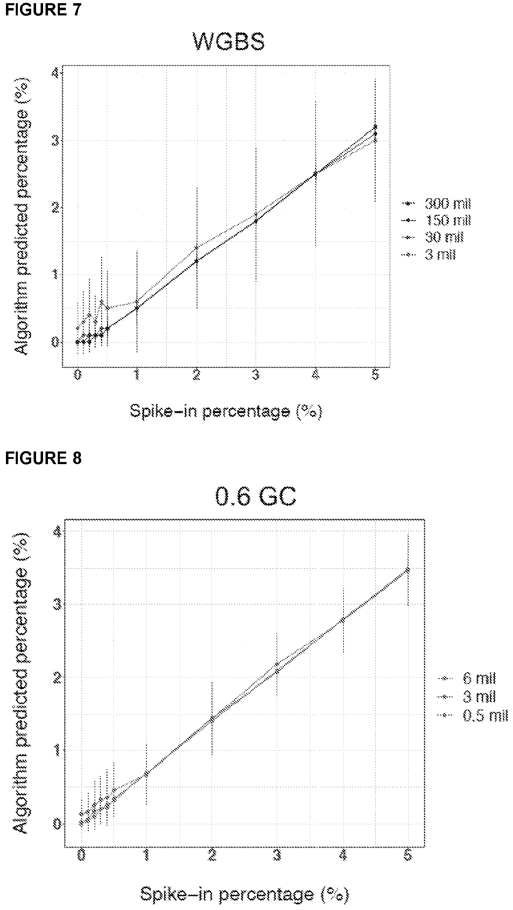

**Figure 7** shows spike-in percentage and algorithm predicted value of WGBS *in silico* datasets at different sequencing depths.
**Figure 8** shows spike-in percentage and algorithm predicted value of Heatrich-BS *in silico* datasets at different sequencing depths.

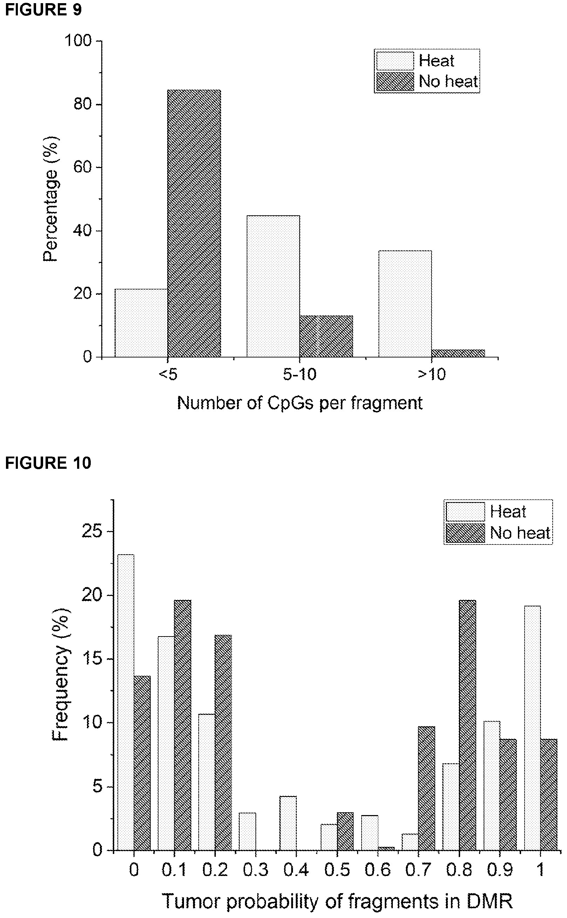

**Figure 9** shows the number of CpGs per fragment with (left bar) and without (right bar) heat denaturation.
**Figure 10** shows the tumor probability of circulating DNA fragments with (left bar) and without (right bar) heat denaturation.

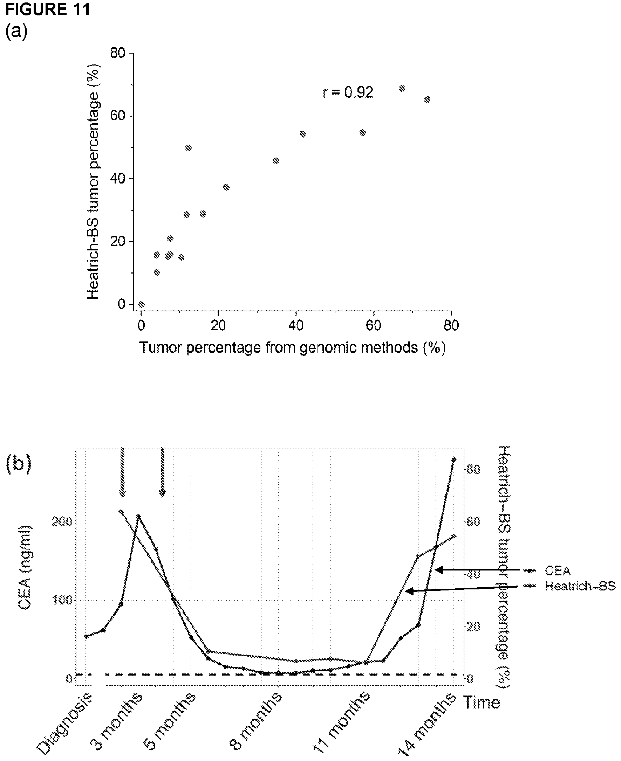

**Figure 11a****-h** shows application of Heatrich-BS on patient cfDNA samples. **(a)** Tumor fractions predicted by genomic methods and Heatrich-BS for patient cfDNA samples. High degree of concordance (Pearson r = 0.92) was achieved between the tumor percentages obtained through the two methods. **(b)** Longitudinal tumor monitoring of Patient 1. XELOX (left arrow) and FOLFIRI-Cetuximab (right arrow) treatment introduction points are marked with arrows. **(c)** Longitudinal tumor monitoring of Patient 2. 5 FU/Oxaliplatin (left arrow) and Irinotecan (right arrow) treatment introduction points are marked with arrows. **(d)** PCA analysis of Patients 1 and 2. PC1 separates patients, with patient 1 on the left of the vertical broken line, while PC2 separates along tumor burden. **(e)** Longitudinal monitoring of 14 CRC patients with Heatrich-BS tumor fraction, SLD (sum of longest diameter) and CEA measurements. CEA positive and negative is indicated by vertical black and grey lines, respectively. Heatrich-BS positive and negative is indicated by filled circles and open circles, respectively. Tumor fraction is represented by the size of the dots. Bottom panel: Cancer detected from both CEA and Heatrich-BS. Middle panel: CEA failed to detect cancer at multiple timepoints. Top panel: Earlier detection of cancer recurrence by Heatrich-BS compared to CEA. **(f)** Heatrich-BS tumor fraction, CEA and SLD values for Patient 357, which shows Heatrich-BS tumor fractions increasing before CEA values. CEA, Heatrich-BS tumor fractions and SLD values are represented by triangles, circles and squares respectively. Open symbols indicate values below threshold. **(g)** Heatrich-BS tumor fraction, CEA and SLD values for Patient 507, which shows Heatrich-BS tumor fractions being informative even when CEA values are uninformative. CEA, Heatrich-BS tumor fractions and SLD values are represented by triangles, circles and squares respectively. Open symbols indicate values below threshold. **(h)** Distribution of Heatrich-BS tumor fractions and CEA values across different tumors sizes. Heatrich-BS tumor fractions produce a more linear relationship with SLD compared to CEA values. Dashed line indicates cancer detection threshold of CEA (5.3 ng/mL).

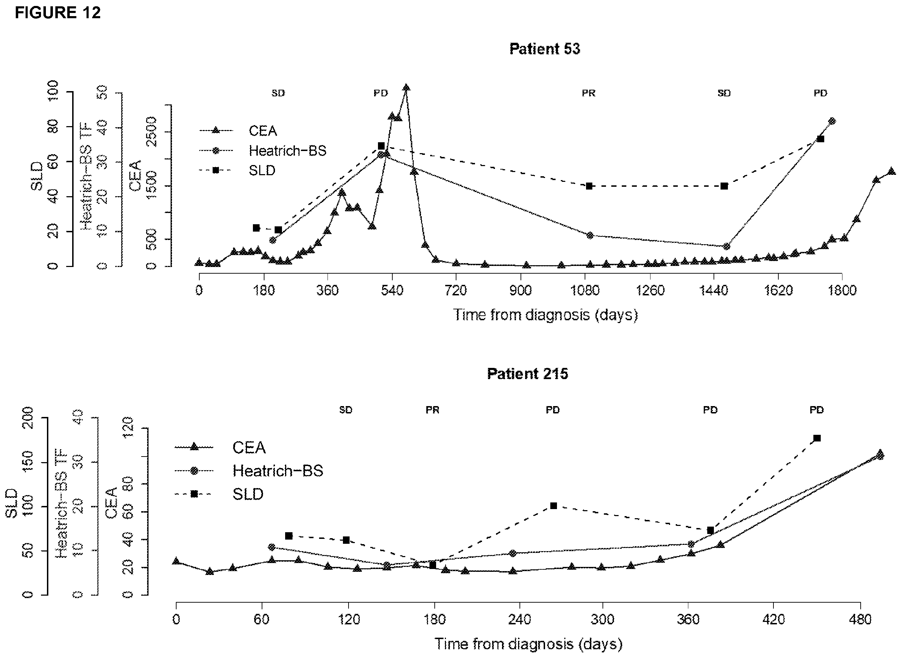

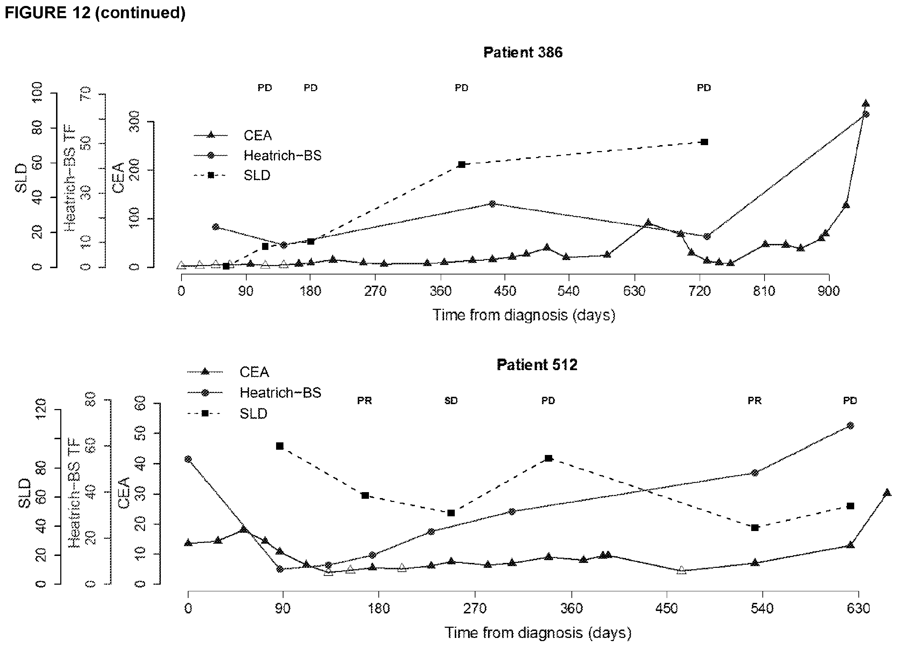

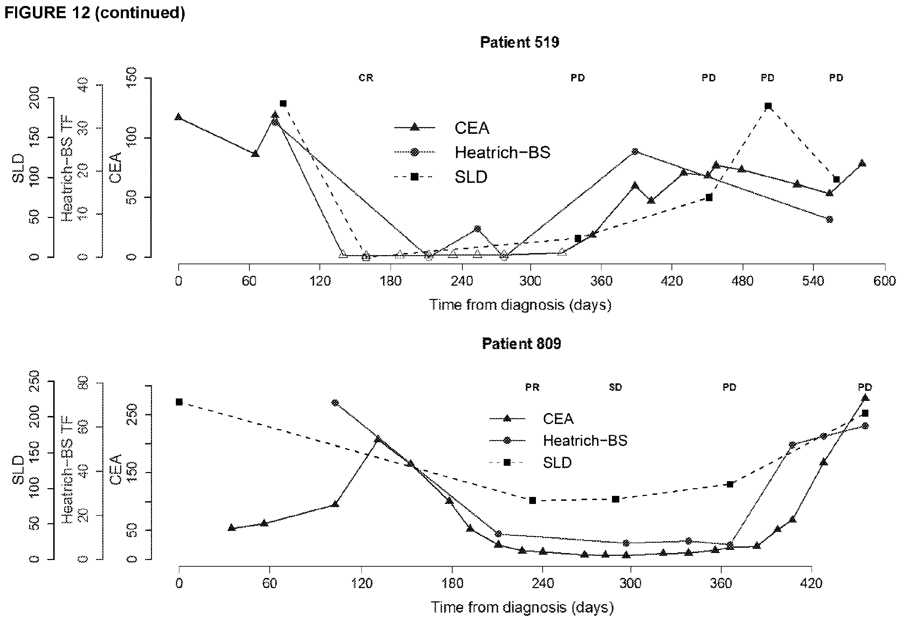

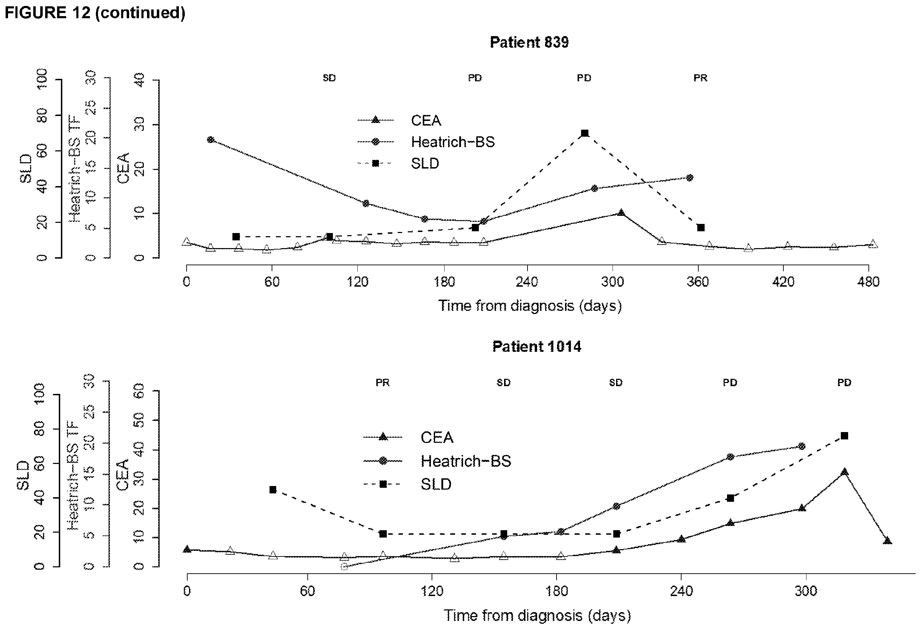

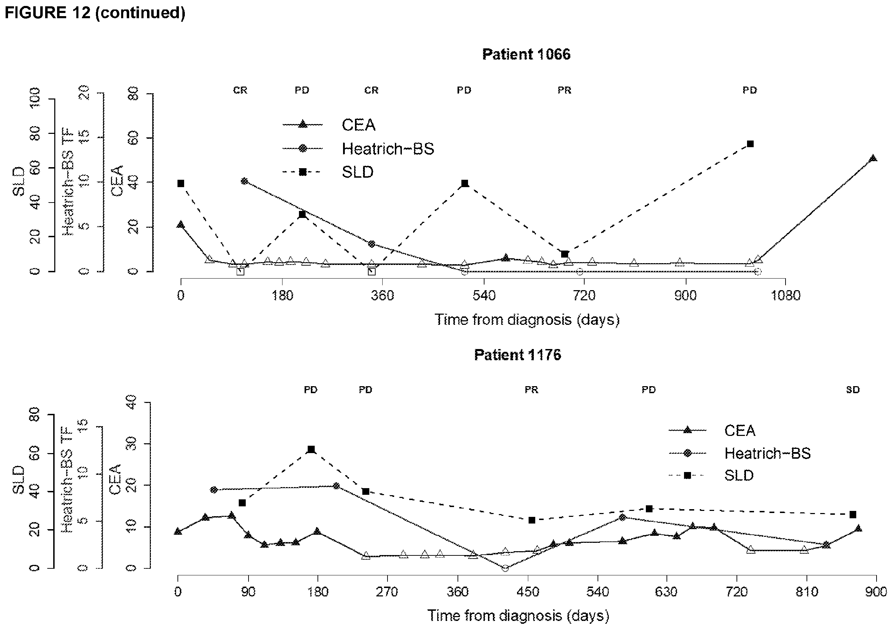

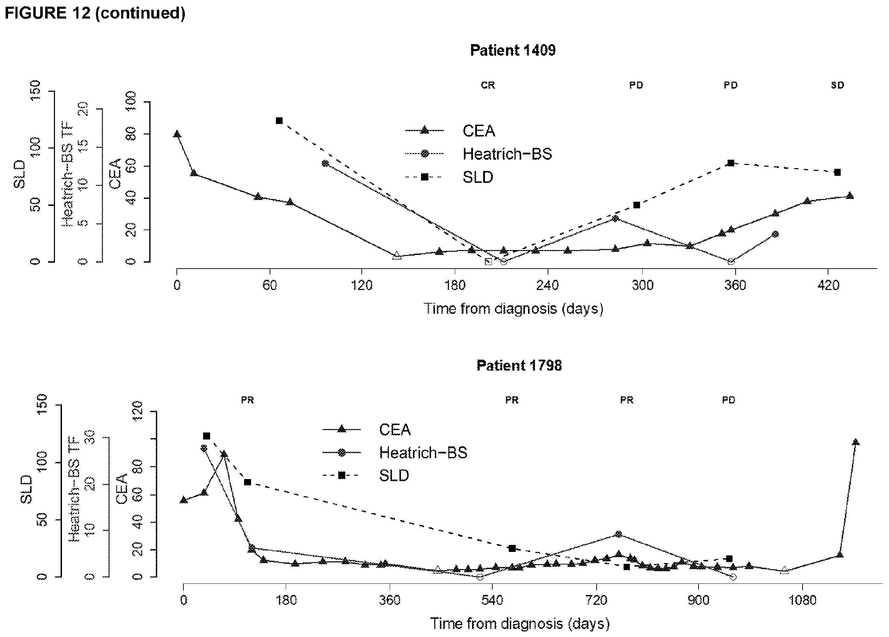

**Figure 12** shows the trend of Heatrich-BS tumor fractions, CEA levels, quantitative tumor measurements (SLD) and disease status across different timepoints of 14 CRC patients. CEA, Heatrich-BS tumor fractions and SLD values are represented by triangles, circles and squares respectively. Open symbols indicate values below threshold.

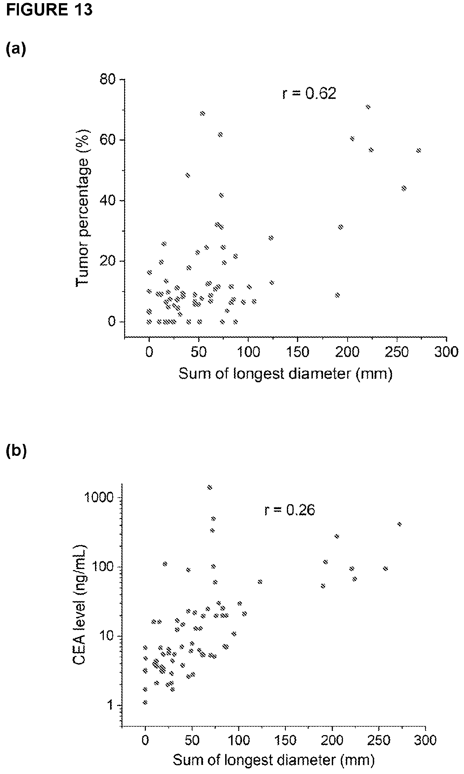

**Figure 13a****-b** show the comparison of quantitative SLD values with orthogonal tumor measurements. **(a)** Relationship between Heatrich-BS tumor fractions and SLD measurements at individual timepoints. Pearson r = 0.62. **(b)** Relationship between SLD measurements and CEA level at individual timepoints. Pearson r = 0.26.

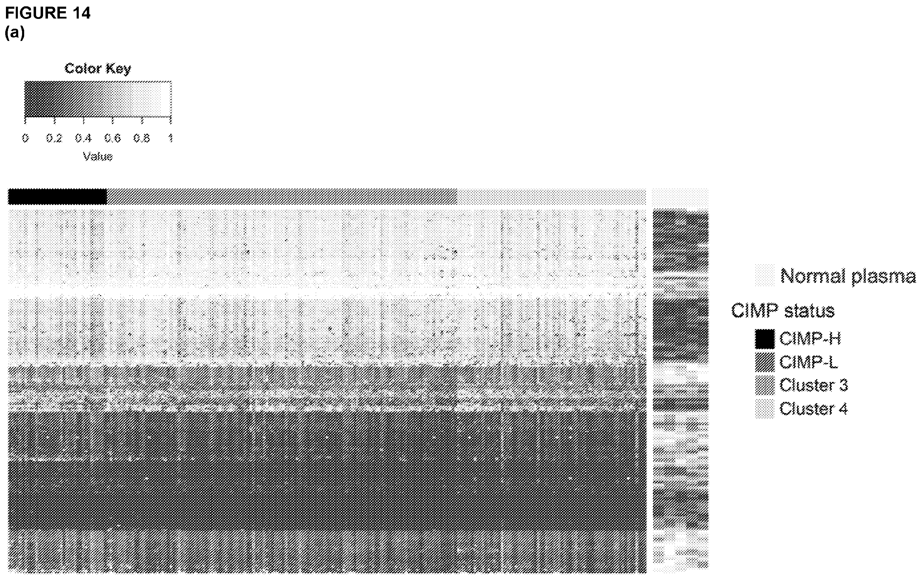

**Figures 14a****-f** shows the characterization of patient cfDNA using Heatrich-BS. **(a)** Methylation status of TCGA CRC samples and normal plasma at 635 CpG markers identified for CIMP subtype classification. **(b)** Methylation score of 233 CRC tissues with CIMP annotation in TCGA. Cut-off thresholds to distinguish different CIMP subtypes was determined by a decision tree classifier. **(c)** Raw and corrected methylation score of simulated cfDNA with different tumor fractions and methylation subtypes. Deconvolution removes the effect of tumor fraction on raw methylation score calculated from cfDNA. **(d)** Corrected methylation scores of different CIMP subtypes and tumor fraction simulated samples with decision tree classifier thresholds for CIMP classification. Corrected methylation scores are concordant across tumor fractions more than 10% for different CIMP clusters. **(e)** Corrected methylation score for patient cfDNA samples with tumor fractions above 10%. **(f)** Methylation status of TCGA CRC samples and normal plasma at DMRs identified for tumor fraction determination. These DMRs are incapable of distinguishing the CIMP subtypes.

### DETAILED DESCRIPTION OF THE INVENTION

Bibliographic references mentioned in the present specification are for convenience listed at the end of the examples.

### Definitions

Certain terms employed in the specification, examples and appended claims are collected here for convenience.

It must be noted that as used herein and in the appended claims, the singular forms "a", "an", and "the" include plural reference unless the context clearly dictates otherwise.

As used herein, the term "comprising" or "including" is to be interpreted as specifying the presence of the stated features, integers, steps or components as referred to, but does not preclude the presence or addition of one or more features, integers, steps or components, or groups thereof. However, in context with the present disclosure, the term "comprising" or "including" also includes "consisting of". The variations of the word "comprising", such as "comprise" and "comprises", and "including", such as "include" and "includes", have correspondingly varied meanings.

The terms "Heatrich" and "Heatrich-BS" are herein used interchangeably as shorthand to describe the method of the invention, being heat enrichment for bisulfite sequencing.

The term "subject" is herein defined as vertebrate, particularly mammal, more particularly human. For purposes of research, the subject may particularly be at least one animal model, e.g., a mouse, rat and the like. In particular, for treatment of cancer, the subject may be a human.

The term "treatment", as used in the context of the invention refers to ameliorating, therapeutic or curative treatment.

A person skilled in the art will appreciate that the present invention may be practiced without undue experimentation according to the methods given herein. The methods, techniques and chemicals are as described in the references given or from protocols in standard biotechnology and molecular biology textbooks. Standard molecular biology techniques known in the art and not specifically described were generally followed as described in Sambrook and Russel, Molecular Cloning: A Laboratory Manual, Cold Springs Harbor Laboratory, New York (2001).

### EXAMPLES

### Methods

### Generating sheared DNA

K562 cells (ATCC^{®} CCL-243^{™}) were cultured in high glucose Dulbecco's modified Eagle's medium (DMEM) (Gibco) supplemented with 10% Fetal Bovine Serum (FBS) (Gibco) and 1% penicillin-streptomycin (Gibco). Genomic DNA was extracted from cultured K562 cells using the DNeasy Blood and Tissue Kit (Qiagen). The extracted gDNA was fragmented using the LE220 Focused ultrasonicator (Covaris) at the following settings: 450W peak incidence power, 30% duty factor, 200 cycles per burst for 420 sec. The fragmented DNA was size selected for 100-200 bp fragments using BluePippin 2% agarose cassette (Sage Sciences).

### Patient recruitment and Extraction of cfDNA from patient blood samples

Colorectal cancer patients were recruited at the National Cancer Centre Singapore under studies 2018/2795 and 2019/2401 approved by the SingHealth Centralised Institutional Review Board. From these patients, blood specimens and tumor specimens were collected where possible and consented for. Blood samples from healthy individuals were collected under study 2012/733/B. Retrospective review of medical records was performed to collect clinicopathological details such as patient demographics, tumor staging, serum CEA and mutational status from clinical testing where available (Table 1).

**Table 1: Clinicopathological features of 14 longitudinal CRC patients**

| **Characteristic** | **Number (n=14)** |
|---|---|
| Median age at diagnosis (years) | 55 (40-72) |

| **Gender** | |
|---|---|
| Male | 9 |
| Female | 5 |

| **Ethnicity** | |
|---|---|
| Chinese | 14 |

| **Sidedness** | |
|---|---|
| Left | 9 |
| Right | 5 |

| **Stage at Diagnosis** | |
|---|---|
| IIIB | 1 |
| IVA | 10 |
| IVB | 3 |

| **MSS/MSI** | |
|---|---|
| MSS | 14 |

| **Mutations (Clinical test)** | |
|---|---|
| KRAS p.G12D | 4 |
| KRAS p.G13D | 1 |
| NRAS p.Q61R | 1 |
| TP53 p.R342* | 1 |

To assess the sensitivity of Heatrich-BS for tumor monitoring in comparison to CEA measurements, patients included those whose CEA measurements were informative or uninformative of disease progression. All plasma was separated from whole blood collected in EDTA tubes within 2 h of venipuncture via centrifugation at 10 min x 300 g and 10 min x 9730 g, and subsequently frozen at -80 °C. Cell-free DNA was extracted using the QiaAmp Circulating Nucleic Acids kit (Qiagen) as per manufacturer's protocol.

### Heatrich-BS protocol

5-10 ng of cfDNA was used as input for the Heatrich-BS protocol. Library preparation was done using KAPA Hyper Prep Kit (Kapa Biosystems). 1.4 µl of End Repair & A-tailing buffer (Kapa Biosystems) and 0.6 µl of End Repair & A-tailing enzyme mix (Kapa Biosystems) was added to 10 µl of input DNA, and incubated at 20°C for 30 min, 65°C for 30 min. Following this, the sample was heated at 88°C for 5 min, and immediately placed on ice. The sample was then topped up with 6 µl of Ligation buffer (Kapa Biosystems), 2 µl of DNA Ligase (Kapa Biosystems), 1 µl of nuclease-free water and 1 µl of 750 nM adapter (Kapa Biosystems). For no-heat controls, 1 µl of 1.5 µM adapter (Kapa Biosystems) was used instead. After adding these reagents, the sample was incubated at 25°C for 1 hour and then cleaned up by performing two rounds of 1.2x SPRI Select (Beckman Coulter). The sample was then subject to bisulfite conversion following the recommended protocol of Zymo EZ DNA Methylation-Gold kit (Zymo Research). The bisulfite converted DNA was amplified for 15 cycles using Pfu Polymerase (Agilent), cleaned up using 1.2x SPRI Select (Beckman Coulter) and re-amplified (10 cycles) using KAPA Hyper Hot-Start Polymerase (Kapa Biosystems) until plateau was reached. The amplified sample was cleaned up using 1.2x SPRI Select (Beckman Coulter), size selected for 190-400 bp fragments using 2% agarose Bluepippin kits (Sage Sciences), quantified using Kapa Library quantification kits (Kapa Biosystems) and sequenced using MiSeqv3 150 cycle kit or Novaseq (Illumina). Pair end sequencing of 75 bp each was performed.

### Heatrich-BS Analysis Pipeline

Fastqc [Andrews, S. FastQC: a quality control tool for high throughput sequence data. (2010)] was used to check the quality of the pair-end reads generated by a MiSeq sequencer (Illumina). After adapter trimming using Cutadapt Software [Martin., M. EMBnet.journal 17, 10-12 (2011)], the reads were aligned to the hg38 human genome using Bismark Software [Krueger, F. & Andrews, S. R. Bioinformatics 27, 1571-1572 (2011)]. The aligned reads were deduplicated using Picard tools [Broad Institute. Picard Tools. (2018)], following which the Bismark methylation extractor was used to obtain per-base methylation status of each fragment.

### GC content calculation

To calculate the GC content of each fragment, the forward and reverse reads were aligned separately, and then combined to generate a single coordinate range encompassing the entire fragment. The coordinates of the fragment were then used to obtain its sequence from the reference genome. For each fragment, the GC content was defined as the number of Cs and Gs, divided by the total length of the fragment. Percentage of reads in CGI was defined as the proportion of sequenced reads that coincided with hg38 CpG island annotation from UCSC.

### Tumor fraction determination algorithm

The tumor fraction determination algorithm has three major steps:

### Step 1: Identifying the differentially methylated clusters

To identify differentially methylated clusters for tumor-specific cfDNA detection, normal plasma whole genome methylation data [Sun, K. et al. Proc. Natl. Acad. Sci. U. S. A. 112, E5503-E5512 (2015)] and colorectal adenocarcinoma (COAD) methylation array from TCGA was used. 23 WGBS datasets for normal plasma and 353 450k methylation array datasets from TCGA were used for cluster generation. The TCGA methylation values were extrapolated to ± 100 bp of each probe site. To ensure selection of only consistent sites, only methylation values with a standard deviation less than 0.4 between the various sample in that class were chosen, to ensure confidence for the reference. DMRfinder Software [Gaspar, J. M. & Hart, R. P. BMC Bioinformatics 18, 1-8 (2017)] was used to identify differentially methylated clusters. Within these clusters, sites with a 0.5 difference in methylation were selected.

### Step 2: Using a bimodal distribution to calculate the class-specific probability of each site.

Using the generated reference, a normal and tumor class-specific must be assigned to each assayed fragment. Since methylation values are binary, the average methylation value observed in the reference is a proportional combination of the unmethylated and methylated reads. As such, a bimodal distribution can accurately represent the proportional methylation status of the reference. For every site in the reference, the contribution from the unmethylated and methylated mode (0 and 1) was calculated. The relative contributions of each mode in the two classes were used to assign class-specific probabilities for the methylation values in the assayed fragment. In this way, a bimodal reference was used to assign normal or tumor probability values to each site assayed.

### Step 3: Using maximum likelihood estimation to predict the tumor fraction of the sample.

After assigning class-specific probabilities to each fragment, the fraction of fragments that come from the tumor must be enumerated. The tumor-derived cfDNA in a sample, also known as tumor fraction, can be denoted as *θ*, where 0 ≤ *θ* < 1. To estimate the tumor fraction *θ*, a maximum likelihood estimation approach and grid search, adapted from CancerDetector [Li, W. et al. Nucleic Acids Res. 46, e89 (2018)], was used to calculate the global tumor fraction (*θ_{g}*) for each sample. The determined tumor purity correction factor (y) of 0.057 is then applied to the raw tumor fraction to generate the final tumor fraction.

### GC content analysis in DMRs

The human genome was split into 200 bp tiling windows and the GC content of each window was calculated. Windows with GC content exceeding 60% were used as a theoretical representation of Heatrich output. To investigate the relationship between GC content and CpG density, we used a random sequence generator to create half a million 200 bp fragments. The number of G+C bases (GC content) and number of CG dinucleotides (CpG content) was calculated as a fraction of the total length. To calculate the number of DMRs per 1000 fragments, we first generated DMRs for each cancer using the earlier mentioned approach (Step 1 of tumor fraction determination). We then utilized random 1000 fragments generated from a plasma cfDNA dataset subject to different GC content thresholds and counted the number of DMRs of each cancer that was detected. To generate the number of DMRs covered by different numbers of total sequencing reads, a similar approach was utilized where different datasets were sub-sampled to the required number of reads and the number of fragments that contained DMRs were counted. For cfRRBS, all fragments with Mspl cut-sites between 20 and 160 bp were used as theoretical data.

### Tumor burden estimation by whole genome or targeted sequencing

DNA libraries were prepared using the Kapa Hyper Prep Kit (Kapa Biosystems) and sent for whole genome sequencing or targeted sequencing. Hybridization capture was done for targeted sequencing using an IDT Xgen Custom Panel of 101 cancer genes and reagents as per manufacturer's instructions. Sequencing was performed on an Illumina Hiseq4000 (2 × 150 bp paired end reads). Tumor fraction estimation from whole genome sequencing data was carried out using the ichorCNA algorithm [Adalsteinsson V. A. et al. Nat Commun 8 (2017)]. Variant calling from targeted sequencing data was performed using MuTect Software [Sougnez, C et al. Nat Biotechnol 31:213-219 (2013)] with the tumor fraction estimation being the mean Variant Allele Frequency of 7 known colorectal cancer hotspots (KRAS, NRAS, BRAF, EGFR, APC, TP53, PIK3CA) present in a particular sample.

### Tumor measurements and disease status classification

For each profiled timepoint, the nearest available CT scan image was retrieved from the patient's clinical records. Each lesion on the scan was measured in 2 dimensions (maximum width and maximum length). Indeterminate lesions were not measured. For each timepoint, the Sum of the Longest Diameter (SLD) was determined, providing a representation of the total tumor load present at the timepoint. To ensure consistency, all measurements were carried out by the same clinician. Disease classification for each timepoint was carried out according to the following criteria; Complete Response: disappearance of all lesions; Partial Response: ≥ 30% decrease in the SLD of the lesions compared with the SLD of the previous measured timepoint; Progressive Disease: ≥ 20% increase of at least 5 mm in the SLD of the lesion compared with the SLD of the previous measured timepoint OR the appearance of new lesions >10 mm in diameter; Stable Disease: Neither PR, PD nor CR.

### Subtype classification

In order to identify marker sets for CIMP subtype classification, we utilized the data and annotations from The Cancer Genome Atlas Network's publication [Muzny, D. M. et al. Nature 487:330-337 (2012)]. We selected CpG sites with a minimum standard deviation of 0.25 and showed distinct methylation patterns in the different CIMP subtypes. Since Heatrich-BS disproportionately enriches for CpG-rich regions, we further restricted the marker list to CpGs that were covered by at least 50 Heatrich-BS samples. To determine the threshold values to distinguish CIMP clusters, we utilized the Decision Tree classifier software from KNIME [Berthold, M. R. et al. Studies in Classification, Data Analysis, and Knowledge Organization, GfKL (2007)]. The TCGA dataset with CIMP classification was split 70-30 for training and testing.

To perform CIMP classification of cfDNA samples, a raw methylation score across the marker sites was calculated for each sample. The Methylation Score, defined as the average of methylation values of the 635 loci, was used to summarize the degree of methylation in CIMP loci. To estimate the Methylation Score of the underlying tumor in cfDNA, we note that *M_{cfDNA} = θMₜᵤₘₒᵣ +* (1 - *θ*)*Mₙₒᵣₘₐₗ₋ₚₗₐₛₘₐ.* Substituting *Mₙₒᵣₘₐₗ₋ₚₗₐₛₘₐ* (Methylation Scores from cfDNA of healthy plasma) and *θ* (the tumor fraction calculated from Heatrich-BS), the Methylation Score of the underlying tumor (*Mₜᵤₘₒᵣ*) can be estimated. To calculate *Mₙₒᵣₘₐₗ₋ₚₗₐₛₘₐ*, samples with negative methylation scores or fewer than 100 reads falling into marker sites were excluded.

### Data sources

In order to compare our assay with existing methods, we obtained the following data from NCBI GEO: RRBS (SRR222486), cell-free RRBS (GSM2090507). cfDNA WGBS data for algorithm development and validation was obtained by request from the EGA database (EGAS00001001219). Colorectal cancer tumor WGBS data was obtained from NCBI GEO (SRR1035745). Illumina 450k methylation array data for different cancers was obtained from The Cancer Genome Atlas (TCGA) data repository.

### EXAMPLE 1:

### Enrichment of CpG-rich DMRs using GC content

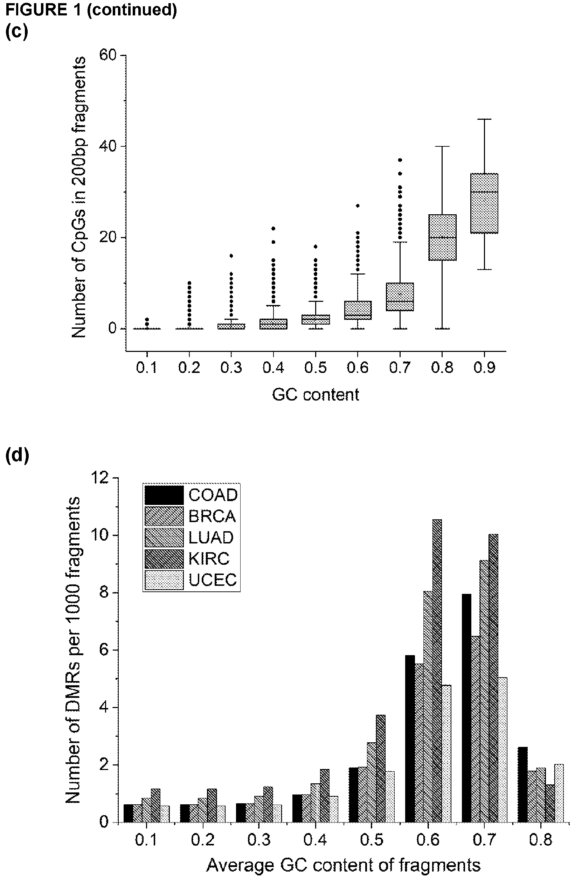

The sequence content in the human genome is highly non-uniform. Long stretches of CpG poor regions are punctuated by short stretches of CpG-dense region that coincide with important gene regulatory elements such as promoters. These CpG-dense regions are often differentially methylated between tissues and disease such as cancer [Guo, S. et al. Nat. Genet. 49, 635-642 (2017)]. We use DMRfinder Software [Gaspar, J. M. & Hart, R. P. BMC Bioinformatics 18, 1-8 (2017)], a popular software, to identify DMRs between colorectal cancer (CRC) tissue and healthy plasma and found that nearly 45% of the DMRs lie within CGls (Fig. 1a), which originates from only 1% of the genome (Fig. 1b). As such, it is of value to focus on this small fraction of CpG-rich genome for epigenetic profiling. The traditional approach to enrich for CGls is RRBS [Gu, H. et al. Nat. Protoc. 6, 468-481 (2011)], with a single-tube variant termed single-cell RRBS (scRRBS) [Guo, H. et al. Nat. Protoc. 10, 645-59 (2015)] for low input samples. However, this approach is still limited in utility for enrichment of fragmented DNA, as very few fragments can meet the requirement for a CCGG cut site on both ends. For example, one study that uses scRRBS to assay for methylation in cfDNA, henceforth referred to as cell-free RRBS, has only 6.4% of the reads in CGls [Guo, S. et al. Nat. Genet. 49, 635-642 (2017)]. While there is no known means to physically enrich for CpG-dense DNA, it is long known that the G+C content of a double-stranded DNA fragment is closely related to its thermal stability. It has been shown that the GC bond in DNA has a binding energy of 25.4 kcal mol⁻¹, which is two times stronger than the AT bond, with a binding energy of 12.4 kcal mol⁻¹. As the presence of a CpG dinucleotide in a fragment adds 2 GC bonds to the duplex, whether effective selection of CpG-dense fragments can be achieved by selection of GC-rich fragments was tested. To check this hypothesis, the GC content and number of CpGs in each fragment in a sample of 0.5 million 200 bp fragments of the human genome were calculated (Fig. 1c). The excellent correlation between GC content and number of CpGs suggests that GC content can be used to enrich for CpG-rich fragments. Fragments with GC content greater than 0.6 constitutes only 2.5% of the genome, but disproportionately includes 85% of CGls and 58% of our identified DMRs. We further verified the relationship between different cancer-specific DMRs and GC content (Fig. 1d). Selection of fragments above 0.6 GC content affords nearly 8-fold enrichment in proportion of reads in DMRs across different cancers (COAD: colorectal adenocarcinoma, BRCA: breast invasive carcinoma, LUAD: lung adenocarcinoma, KIRC: kidney renal clear cell carcinoma, UCEC: uterine corpus endometrial carcinoma). Therefore, we established that DMRs of various cancers, which are universally overrepresented in CpG-dense regions, can be effectively enriched with selection of high-GC DNA fragments.

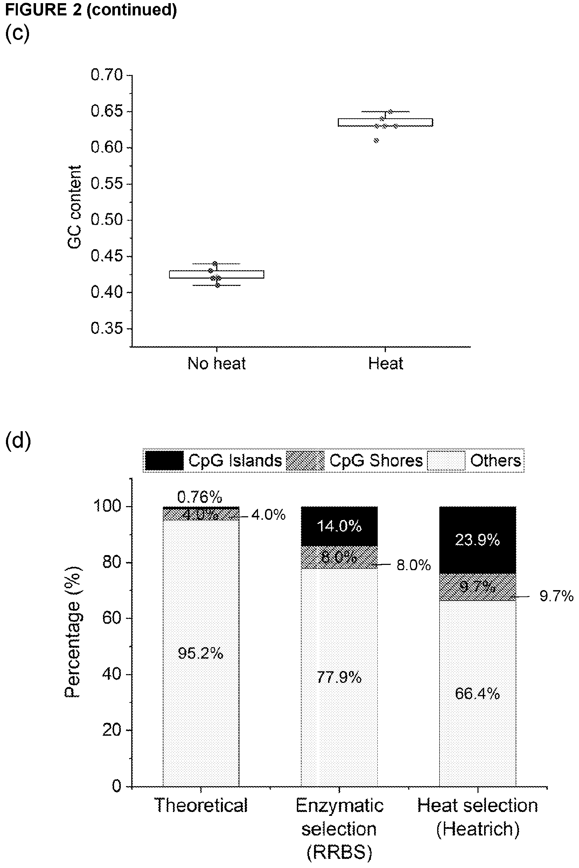

Heatrich uses thermal denaturation to select for DNA fragments with high GC content (Fig. 2a). Fragmented DNA was first end-repaired and A-tailed. Following this, the sample was heated to denature the GC-poor fragments and adapter ligation was immediately performed. The process of adapter ligation allows selection of intact non-denatured GC rich double-stranded fragments, as T4 DNA ligase has a high selectivity for dsDNA [Doherty, A. J. & Wigley, D. B. J. Mol. Biol. 285, 63-71 (1999)]. The selected fragments were bisulfite-converted and subsequently sequenced. We found through empirical optimization experiments using sheared genomic DNA (Fig. 2b) that heating DNA to 88°C immediately prior to adapter ligation yields the best enrichment of reads in CGI (28%) at high GC content (0.63±0.006) compared to the average GC content of the unheated samples (0.42±0.009) (Fig. 2c).

### EXAMPLE 2

### Using heat denaturation to select for GC-rich fragments

Having established that GC content correlates well with CpG density, we then explored the use of heating as a means of GC content selection. The workflow of the Heatrich-BS assay is shown in Figure 2a. Fragmented DNA was prepared for adapter ligation by performing end repair and A-tailing. Following this, the sample was heated to denature the GC-poor fragments and immediately underwent adapter ligation. The process of adapter ligation allows selection of intact double-stranded fragments, as T4 DNA ligase results has a high selectivity for dsDNA due to its high affinity for dsDNA and low affinity for ssDNA [Doherty, A. J. & Wigley, D. B. J. Mol. Biol. 285, 63-71 (1999)]. In this way, adapter ligation can be used to select for the non-denatured GC-rich DNA fragments. The selected fragments were bisulfite converted and subsequently sequenced.

In order to identify the temperature needed to achieve the desired enrichment, a range of temperatures from 75°C to 95°C on sheared genomic DNA were tested (Fig. 2b). The temperature range of 87°C to 90°C had the highest GC content and enrichment of reads in CGI. At higher temperatures, even the fragments with high GC content were denatured, therefore reducing the enrichment in CGls. Further factoring in the alignment rate after bisulfite conversion, we chose 88°C as the denaturation temperature, as this condition had the highest alignment rate along with a sufficiently high GC content (~0.62) and enrichment of reads in CGIs (28%). From multiple experiments performed at the selected temperature, we can see that the GC content of the Heatrich samples was much higher than the average GC content of the genome and unheated samples (Fig. 2c). The consistent GC content and CpG enrichment obtained from multiple experiments also shows the high degree of reproducibility of our assay.

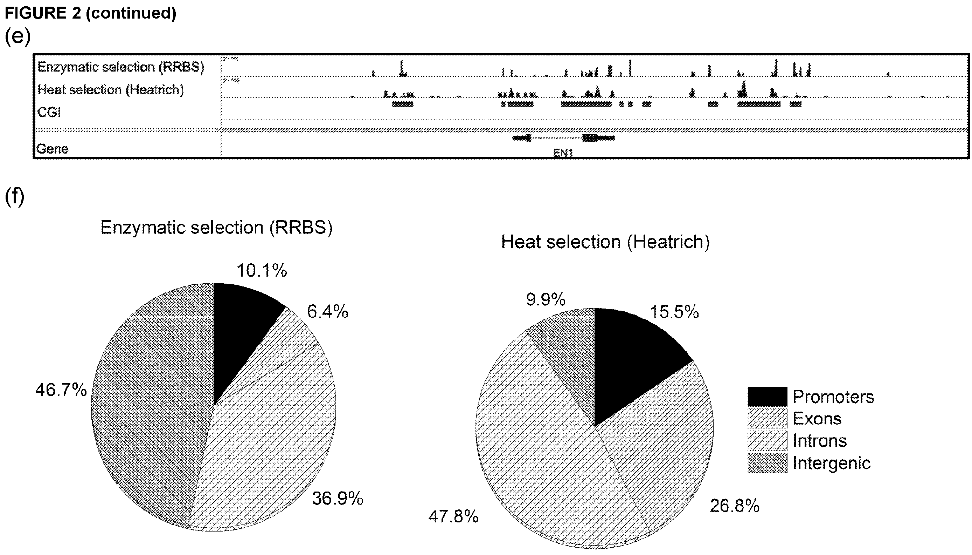

Analysis of the mapping of heat-treated sheared DNA showed that there is indeed a significant enrichment of reads that localized to CpG islands and shores, comparing favorably to the theoretical genomic distribution and even RRBS, the gold standard technique for CGI enrichment (Fig. 2d). The accumulation of reads around CGls was also visualized (Fig. 2e), showing that Heatrich has significant piling up of reads around CGI. More detailed analysis of the genomic distribution of Heatrich reads showed a remarkable similarity to that of standard RRBS (Fig. 2f), suggesting that this non-enzymatic approach can be a viable alternative to RRBS for detailed methylation profiling in important genomic regulatory elements. Notably, Heatrich is independent of restriction enzyme sequence, and will thus robustly profile the same regions even in the presence of restriction site polymorphisms, and could find applications when DNA is already fragmented prior to restriction digestion (e.g. FFPE, degraded DNA, cfDNA) where RRBS is not well-suited.

### EXAMPLE 3

### Enrichment of CpG-rich regions using Heatrich-BS

cfDNA samples are ideally suited for Heatrich-BS due to their fragmented nature, and promising applications for noninvasive disease detection. To test the performance of Heatrich-BS on cfDNA, samples were obtained from cancer patients. Bisulfite treatment was performed on samples with and without heat denaturation. We first visualized the reads obtained from Heatrich-BS and compared it with previously reported cell-free RRBS and WGBS datasets [Guo, S. et al. Nat. Genet. 49, 635-642 (2017)] with similar read counts (Fig. 3a). The reads from cell-free RRBS and WGBS were distributed almost evenly across the genomic region. On the other hand, the vast majority of Heatrich-BS reads piled up at CpG islands and shores, with little reads in the remaining regions. This shows the specificity of Heatrich-BS in selecting only fragments of high GC content. Furthermore, with similar read counts, the average height of Heatrich-BS peaks is appreciably more than that of the other datasets. This shows that Heatrich-BS can obtain higher depth in informative regions using the same number of total reads.

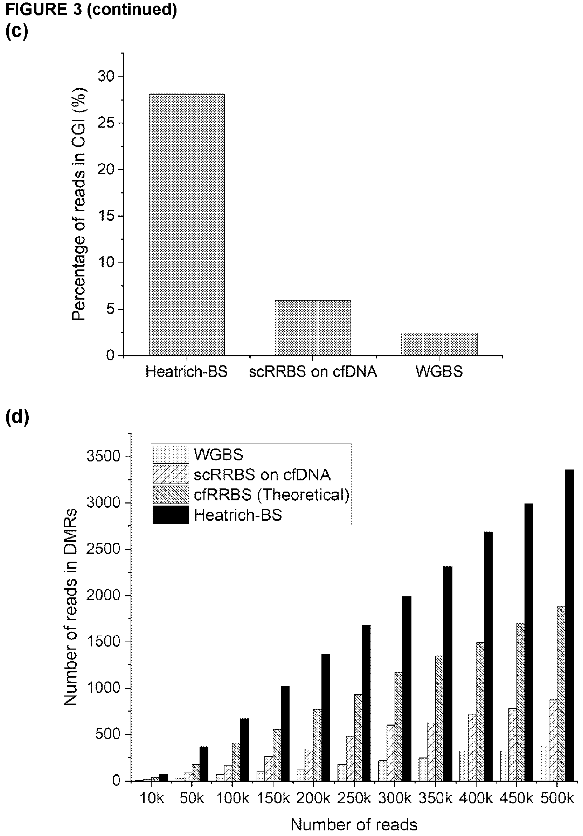

The distribution of sequenced reads from Heatrich-BS and unheated cfDNA were then quantified, and it was seen that the Heatrich-BS samples displayed up to 30-fold enrichment of reads in CGls (Fig. 3b). For 500k unique sequencing reads, almost 30% of Heatrich-BS reads fall within CGls, while only 6% and 2% of cell-free RRBS and WGBS reads are in CGls (Fig. 3c). This demonstrates that Heatrich-BS displays more effective CGI enrichment for fragmented DNA, compared to the performance of cell-free RRBS on similar samples. Furthermore, the number of reads in DMRs were measured for different sequencing reads (Fig. 3d). It is evident that Heatrich-BS has up to 10-fold more reads localizing to DMRs and Heatrich-BS is able to detect up to 10-fold more DMRs compared to the no heat controls (Fig. 4). This would provide higher sensitivity in detecting fragments of tumor origin for the same number of sequencing reads. These figures highlight the strength of Heatrich-BS, which is the effective enrichment of reads in CGI and DMRs, even with fewer sequencing reads.

### EXAMPLE 4

### Estimating the tumor fraction

In order to determine tumor fraction from methylation data, recent studies have utilized a fragment-wise approach as it offers high sensitivity [Guo, S. et al. Nat. Genet. 49, 635-642 (2017); Li, W. et al. Nucleic Acids Res. 46, e89 (2018)]. As such, we adopted a fragment-based approach for our tumor fraction determination algorithm. The workflow of our algorithm is shown in Figure 5a and 5b, and detailed in the methods section.

Using 450k array methylation data from TCGA to generate the colorectal cancer reference has a key pitfall: the resected tumor sample used for the array is often contaminated with normal cells. This contamination has been widely acknowledged [Leary, R. J. et al. Proc. Natl. Acad. Sci. U. S. A. 105, 16224-9 (2008)] and tools have been developed to account for this contamination in somatic variant calling [Sendorek, D. H. et al. BMC Bioinformatics 19, 28 (2018)]. In methylation-based analysis, this contamination could lead to overestimation of the tumor fraction. Therefore, we attempted to identify the contribution of non-neoplastic cells to the colorectal cancer reference, and eliminate its effect on our tumor fraction determination. To this end, we generated a colorectal cancer reference using 23 healthy volunteer cfDNA datasets [Sun, K. et al. Proc. Natl. Acad. Sci. U. S. A. 112, E5503-E5512 (2015)] for the normal and 353k TCGA array datasets for the tumor reference. This reference was then applied to 3 other healthy cfDNA datasets to estimate the tumor fraction. We observed a stable non-zero baseline value (Fig. 6), irrespective of the sequencing depth. Similar observations of non-zero baseline of healthy cfDNA has been reported when tissue samples rather than pure cell populations were used as references. This baseline value is attributed to the contribution of non-neoplastic cells from tumor tissue references and commonly leads to overestimation of the tumor fraction in methylation-based analysis. To identify the contribution of these non-neoplastic cells to the colorectal cancer reference, and eliminate its effect on our tumor fraction determination, we determined a tumor purity correction factor by performing Receiver Operating Characteristic (ROC) analysis of healthy and simulated plasma cfDNA WGBS samples at 0.5% tumor fraction. The specific correction factor (γ) that maximized sensitivity and specificity across multiple sequencing depths (Table 2) was determined for the generated CRC reference. This factor is dependent on the reference used and only needs to be determined once for each reference.

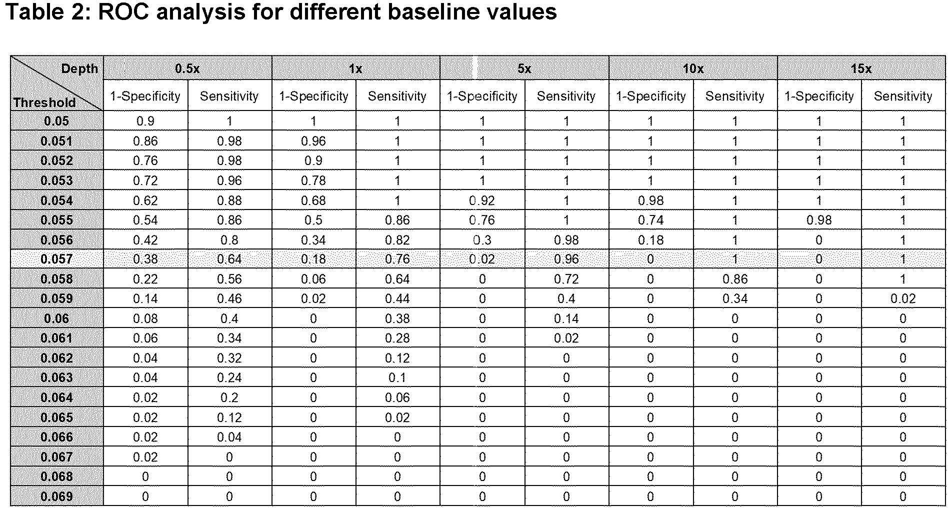

The highlighted threshold of 0.057 was selected as the tumor purity correction factor (y).

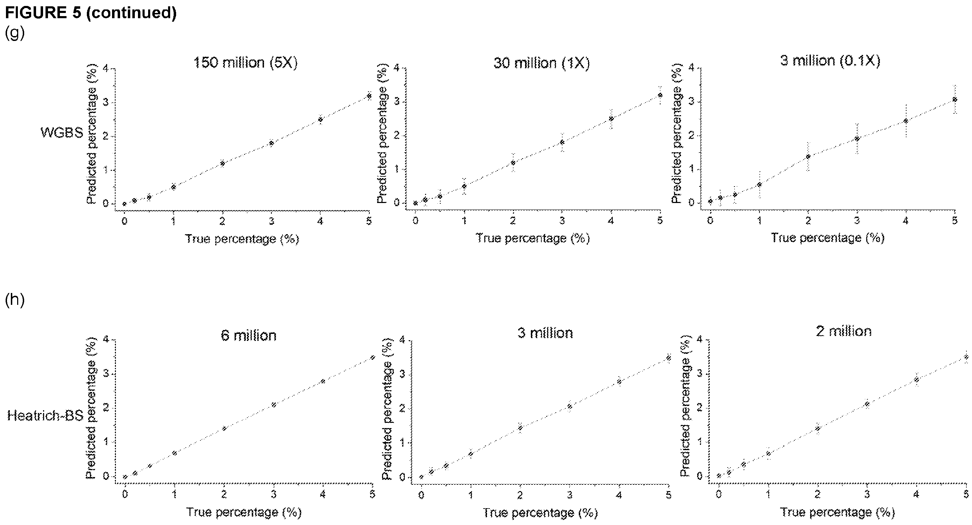

Using the determined correction factor, we tested our algorithm on simulated plasma WGBS cfDNA samples from 0% to 5% tumor fraction, at different sequencing depths (Fig. 5g). At sequencing depths of 5X and 1X, we obtained a high degree of linearity (Pearson correlation >0.99) between the simulated and predicted tumor fraction values while the estimated tumor fraction for the healthy individuals was correctly called as zero. Notably, at 1X depth, where each DMR is covered only once on average, our algorithm can accurately detect the presence of small tumor fractions. This is achieved by aggregation of reads from multiple loci, without requiring high depth at individual DMRs. Despite this improvement, excessively low coverage would lead to limited number of DMRs being interrogated, which would in turn affect the specificity and confidence of tumor calling, as evidenced by the larger variations in the predicted tumor fractions, including a higher likelihood of false positives in healthy cfDNA samples at 0.1X sequencing depth. Using the CpG enrichment offered by Heatrich-BS, the sequencing requirement can be kept low without sacrificing coverage of DMRs. To validate this, we approximated the Heatrich-BS assay by selecting only plasma cfDNA fragments with >0.6 GC content (simulated Heatrich-BS samples). We observed that even using very modest number of total sequencing reads (2-6 million reads), high specificity and tumor calling confidence could be achieved (Fig. 5h). Notably, the tumor fraction prediction from simulated Heatrich-BS samples had much higher specificity and lower variance compared to a similar read count of WGBS samples (0.1X) at 3 million reads. ROC analysis of WGBS and Heatrich-BS for low tumor burden detection in cfDNA (0.5% tumor fraction) showed that the predictive accuracy of Heatrich-BS samples is significantly better than conventional WGBS samples (AUC 0.988 vs 0.547) (Fig. 5i). These results demonstrate that Heatrich-BS and the corresponding algorithm enable accurate tumor DNA detection in cfDNA with significantly lesser sequencing requirement compared to existing methods.

Applying the determined correction factor, we utilized our algorithm to estimate the tumor fraction of 0.1% to 5% spike-in datasets at different depths (Fig. 7). The same analysis was repeated with Heatrich-BS simulated data, using the previously established GC cut-off of 0.6 (Fig. 8). The Pearson correlation between the true and algorithm-predicted spike-in values was 0.999, demonstrating the accuracy and sensitivity of our algorithm.

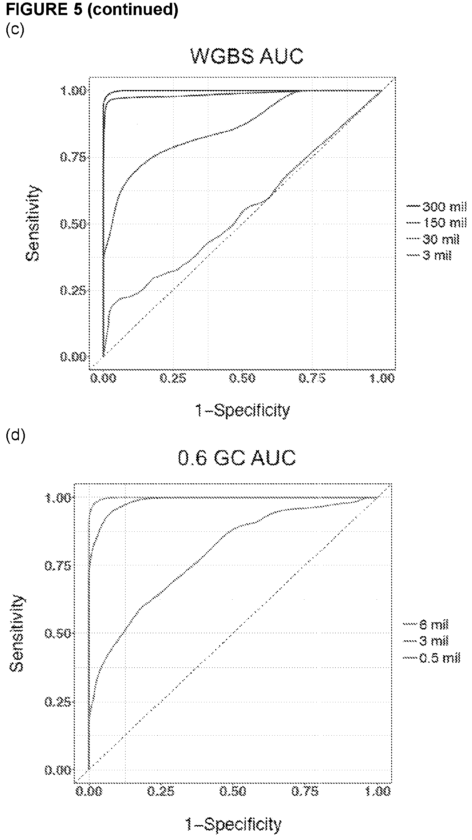

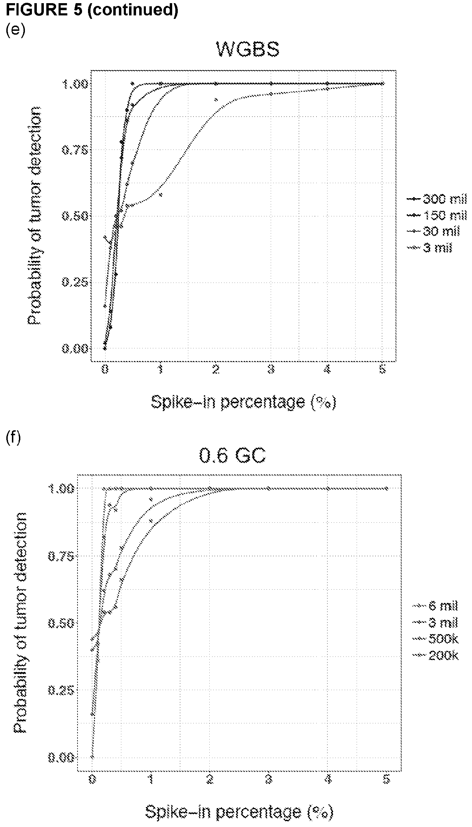

We then visualized the power of our algorithm in detecting 0.5% tumor fraction using different number of total sequencing reads. ROC curve analysis shows that our algorithm is able to achieve 98% predictive accuracy (AUC: 0.984) using 150 million WGBS reads (Fig. 5c). The same accuracy can be achieved with as low as 3 million reads using the Heatrich-BS method (AUC: 0.988) (Fig. 5d). Furthermore, comparing 3 million reads using WGBS and Heatrich-BS, we can see the marked improvement in performance and accuracy using Heatrich-BS (AUC 0.547 vs 0.988). We then analysed the probability of tumor detection at different sequencing depths using WGBS and Heatrich-BS (Figs. 5e & f). At 3 million reads, Heatrich-BS has a 0.82 probability of detecting a 0.2% tumor fraction, while WGBS has only a 0.46 probability of the same. Therefore, this demonstrates that Heatrich-BS can outperform WGBS in accuracy, sensitivity and robustness of tumor prediction.

The main feature of our algorithm is that it is not heavily dependent on the sequencing depth. Existing tumor fraction determination algorithms largely rely on deconvolution [Sun, K. et al. Proc. Natl. Acad. Sci. U. S. A. 112, E5503-E5512 (2015)], which is highly dependent on sequencing depth. To detect a 1% tumor fraction, at least 100x coverage is required. Even fragment-based approaches using software such as CancerDetector [Li, W. et al. Nucleic Acids Res. 46, e89 (2018)] require more than 2x sequencing depth, since their 'confounding marker removal' step is dependent on the coverage of each marker. Therefore, all existing methods to determine tumor fraction requires >2x sequencing depth, which is in excess of 60 million reads. On the other hand, our developed algorithm is not heavily dependent on sequencing depth, as reads from across the genome can be aggregated to determine global tumor fraction. In conjunction with the Heatrich-BS assay, our algorithm can achieve up to 99% predictive accuracy with as few as 3 million reads (Fig. 5c). This further demonstrates the power of our algorithm to enable cost-effective tumor-specific cfDNA detection from liquid biopsy.

### EXAMPLE 5

### Application of Heatrich-BS on cfDNA samples

Finally, we applied Heatrich-BS and the developed algorithm on 5 healthy volunteers and 15 colorectal cancer patient cfDNA samples (2 to 8 million sequencing reads each) and compared the tumor fraction obtained from either whole genome sequencing or deep targeted sequencing (Fig 11a). We observed that Heatrich-BS reads contain more CpGs per fragment (Fig. 9), making each sequenced read more informative. Therefore, there were more fragments with tumor probability higher than 90% or lower than 10% when applying the Heatrich-BS method (Fig. 10), which increases the confidence and accuracy of the tumor fraction called. We then compared our algorithm predicted values to the tumor fraction estimated using the gold standard amplicon sequencing (amplicon-seq) method for mutation detection (Fig. 11a). We obtained a Pearson correlation of 0.92 between the Heatrich-BS and amplicon-seq predicted tumor fractions, proving the accuracy of our method and algorithm even in clinical cfDNA samples.

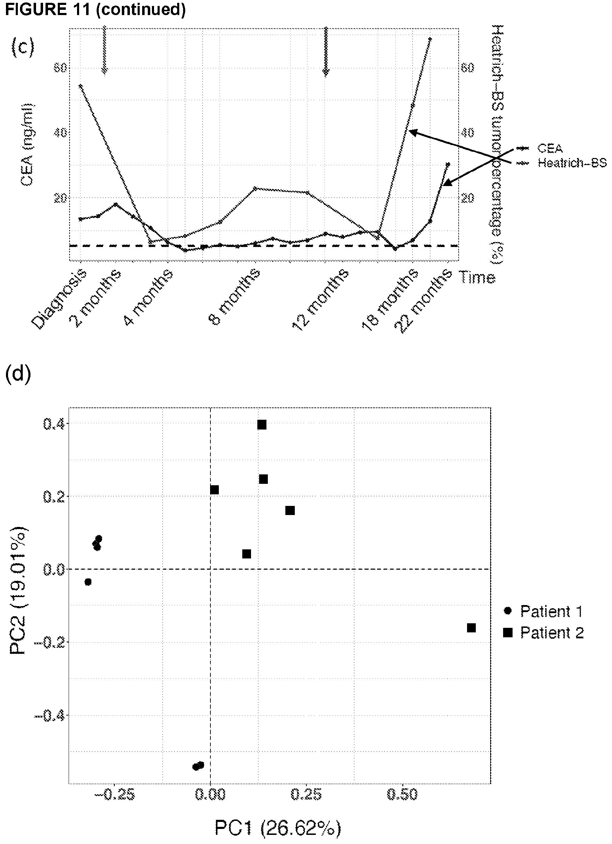

Besides non-invasive cancer diagnosis, liquid biopsy is useful for non-invasive tracking of disease progression. The low cost and high sensitivity for quantitative tumor fraction estimation enabled by Heatrich-BS is attractive for frequent monitoring of patients undergoing treatment and those in remission to detect possibility of relapse. To further validate the applicability of Heatrich-BS in cancer progression monitoring, longitudinal samples were obtained from two colorectal cancer patients at different time points during treatment. Using Heatrich-BS, we were able to obtain tumor percentage values and track the patient's response to treatment (Fig. 11b & c). The trend in tumor percentage predicted by Heatrich-BS is comparable to the CEA values, which is a known biomarker for colorectal cancer diagnosis and monitoring. However, increased CEA level in blood is not specific to colorectal cancer, and has been shown to only have 34% sensitivity and 84% specificity for recurrence monitoring [Wanebo, H., et al., Surg. Gynecol. Obstet. 169, 479-487 (1989)]. Since Heatrich-BS requires fewer reads for tumor percentage prediction, and therefore can be performed at lower cost, it allows more frequent and regular monitoring using this more sensitive method. Furthermore, CEA level only offers one-dimensional data on the level of cancer while Heatrich-BS can offer multi-dimensional data, including a more quantitative measure of tumor burden as well as other information that can distinguish patients (Fig. 11d). It is interesting to note that, on performing Principal Components Analysis (PCA) on the longitudinal patient samples, PC1 separates the patients while PC2 separates along tumor burden. This multi-dimensional information provided by Heatrich-BS can potentially be used to infer subtype classification, drug resistance and other tumor characteristics. Therefore, cost-effective Heatrich-BS can be used to sensitively monitor tumor progression and characteristics in the clinical context.

Current noninvasive surveillance methods for monitoring CRC therapy efficacy and detecting cancer recurrence have their limitations. Radiation exposure and cost limits the frequency at which CT scans can be performed. Serum protein biomarkers such as CEA can be measured frequently, but it lacks sensitivity and specificity [Shinkins, B et al. PLoS One 12 (2017)]. We performed a cost analysis of Heatrich-BS (at 3 million reads per sample) and estimated the assay cost to be less than $30 (Table 3).

**Table 3: Cost of Heatrich-BS assay**

| **Reagent/Step** | **Cost per reaction (USD)** | **Cost per sample* (USD)** |
|---|---|---|
| End-repair, A-tailing, adapter ligation (Kapa HyperPrep Kit)** | 6 | 6 |
| Cleanup (Beckman Coulter SPRIselect) | 20 | 4 |
| Bisulfite conversation (Zymo EZ DNA Methylation-Gold Kit) | 5 | 1 |
| Size selection (Sage Sciences Bluepippin) | 15 | 3 |
| Library QC | 10 | 2 |
| Sequencing (3 million reads) | 13 | 13 |
| Total | - | 29 |

| | | |
|---|---|---|
| *Cost per sample is calculating assuming 5 samples in each pool **Volumes were scaled down, deviating from the kit protocol (refer to Methods) | | |

Due to its simple workflow and low cost, Heatrich-BS has the potential to be a sensitive assay for frequent monitoring of patients undergoing treatment and those in remission to detect possibility of relapse. To validate the applicability of Heatrich-BS in cancer progression monitoring, we further profiled a cohort of 79 samples from 14 CRC patients across their course of treatment, with 5-7 timepoints per patient (Table 1).

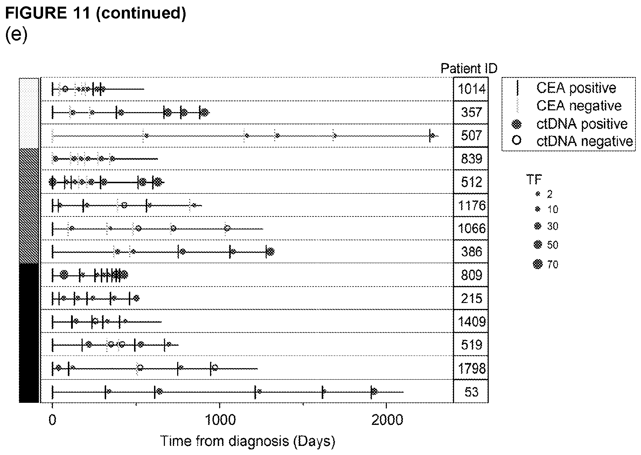

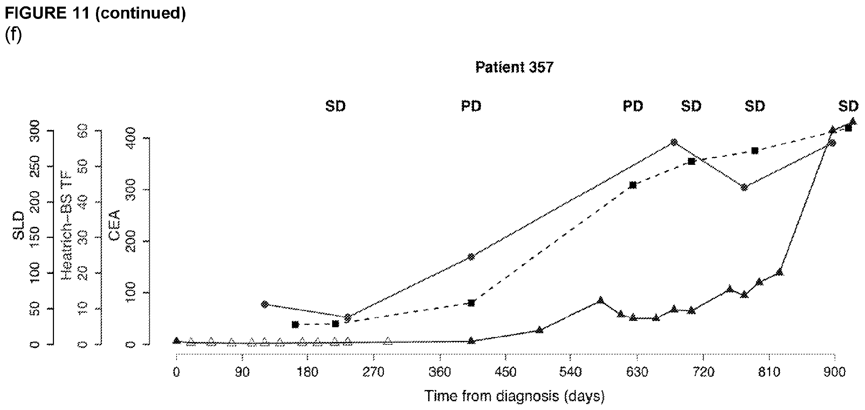

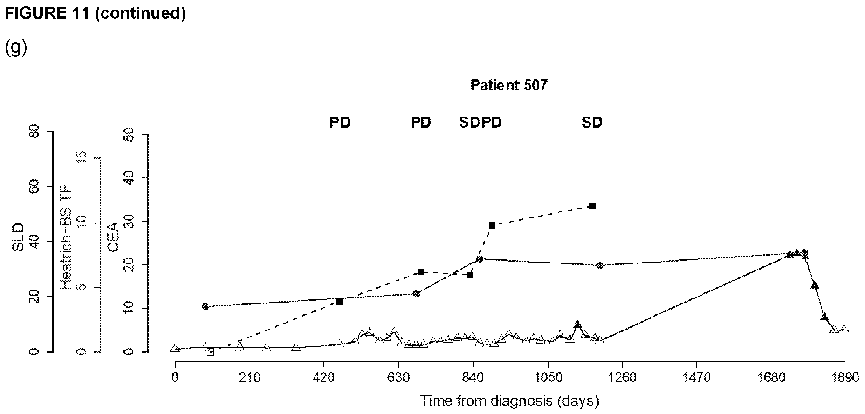

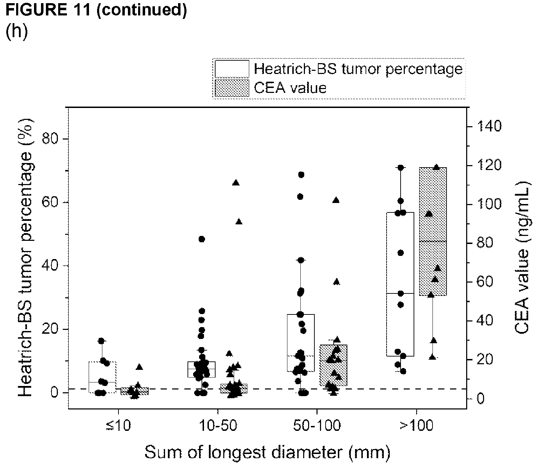

Concurrently, we obtained longitudinal CEA measurements and computed tomography (CT) scans of these patients for benchmarking tumor fraction predictions by Heatrich-BS (Fig. 12). From the aggregated measurements of our cohort, we observed that CEA values do not correlate well with the sum of longest diameter (SLD) of lesions in CT scan (Pearson r = 0.26, Fig. 13b), highlighting the limitation of CEA as a quantitative measurement. Further analyzing the timepoints for each patient, the trend in Heatrich-BS tumor fractions was visualized and compared with CEA status (Fig. 11e). It was seen that Heatrich-BS tumor fractions can outperform traditional CEA measurements in two aspects: (1) Heatrich-BS tumor fractions increase before CEA measurements, enabling earlier detection of tumor recurrence (e.g. Patients 357, 1014, 507) (Fig. 11f). (2) Heatrich-BS can detect tumor burden when CEA cannot, increasing sensitivity of detection (e.g. Patients 357, 1176, 519, 839, 507, 1066, 1014) (Fig. 11g. Despite CEA being the most common test performed for routine monitoring of CRC, we observed that CEA values were non-informative in detecting new or recurrent lesions in 6 of the 14 patients (43%), even when the lesions were visible on CT scans, while Heatrich-BS is concordant with CT scans in 93% patients. 22 of the 70 CEA measurements (31%) correspond to time points when a lesion is detected in CT scan are within normal CEA level limits (<5.3 ng/mL). On the other hand, tumor fraction predictions by Heatrich-BS correlated better to SLD measurements of corresponding time points (Fig. 11h and Fig. 13a Pearson r = 0.62). Fewer measurements (8 time points, 11%) were discordant between detection of tumor via Heatrich-BS (LOD 0.5%) and observation of lesions on CT scans. Therefore, we have demonstrated that Heatrich-BS can accurately track disease progression in patients where traditional CEA measurements are non-informative, establishing this assay as a sensitive and cost-effective non-invasive monitoring tool.

### EXAMPLE 6

### Characterization of tumor methylation subtypes in patient cfDNA using Heatrich-BS

Tumorigenesis can be driven by a myriad of genetic or epigenetic factors, resulting in distinct subtypes within a type of cancer. One common methylation subtype, known as CpG island methylator phenotype (CIMP), is observed in multiple cancers such as CRC, breast cancer, gastric cancer and glioma among others and is characterized by epigenetic instability, where tumor suppressor genes are inactivated by methylation rather than mutation [Mojarad, E. N. Gasrolenterol Hepatol from Bed to Bench 6:120-128 (2013)]. Studies have shown that patients with CIMP-positive tumors have poorer prognosis and shorter overall survival [Juo, Y. Y. et al. Ann Oncol. 25:2314-2327 (2014)], while CIMP-positive CRCs respond better to irinotecan-based regimen rather than oxaliplatin-based regimen [Zhang, X et al. Front Oncol 11 (2021)]. To our knowledge, no existing targeted cfDNA methylation assay is capable of performing cancer methylation subtyping. The untargeted nature of Heatrich-BS provides an opportunity to obtain this additional insight.

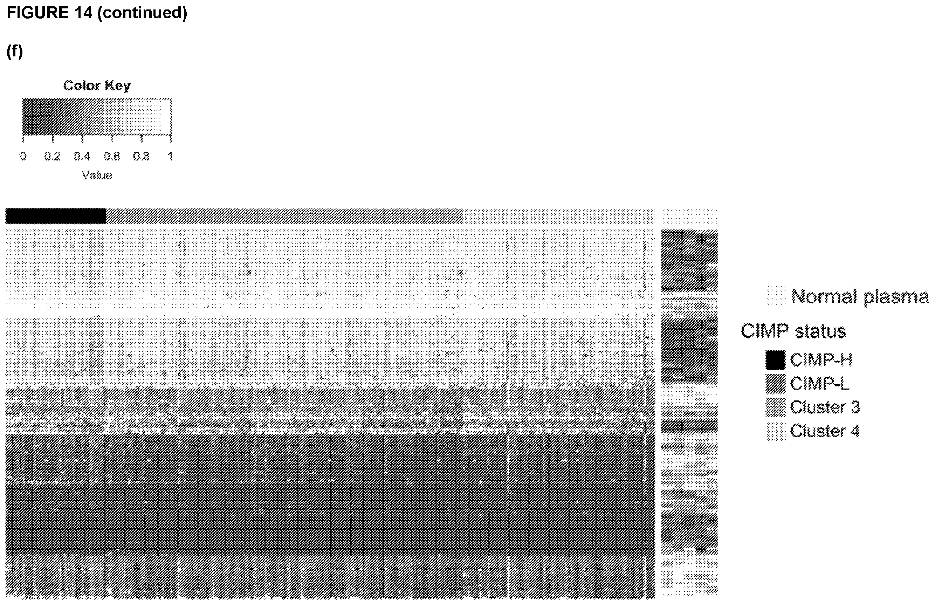

The majority of differentially methylated loci in CIMP are found in CGls that are highly enriched in Heatrich-BS. We found that 41.7% (1121/2686) of the loci used to classify and annotate CIMP status in TCGA CRC samples [Muzny, D. M. et al. Nature 487:330-337 (2012)] are effectively represented in Heatrich-BS (covered in more than 50 samples). We identified a final set of 635 most informative CpGs that can collectively distinguish the different CIMP subtypes (Fig. 14a). On the other hand, methylation profiles of the DMRs used to predict tumor fraction remained invariant across the different CIMP subtypes (Fig. 14f). Interestingly, we noted that there is no overlap between CIMP markers and the DMRs used to predict tumor fraction, indicating that Heatrich-BS regions encompass orthogonal sets of markers that are useful for tumor load quantification and methylation subtype prediction.

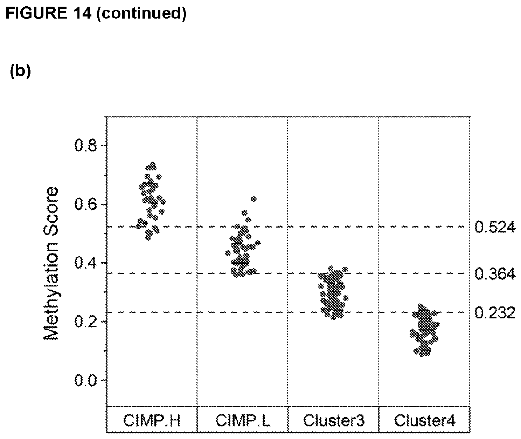

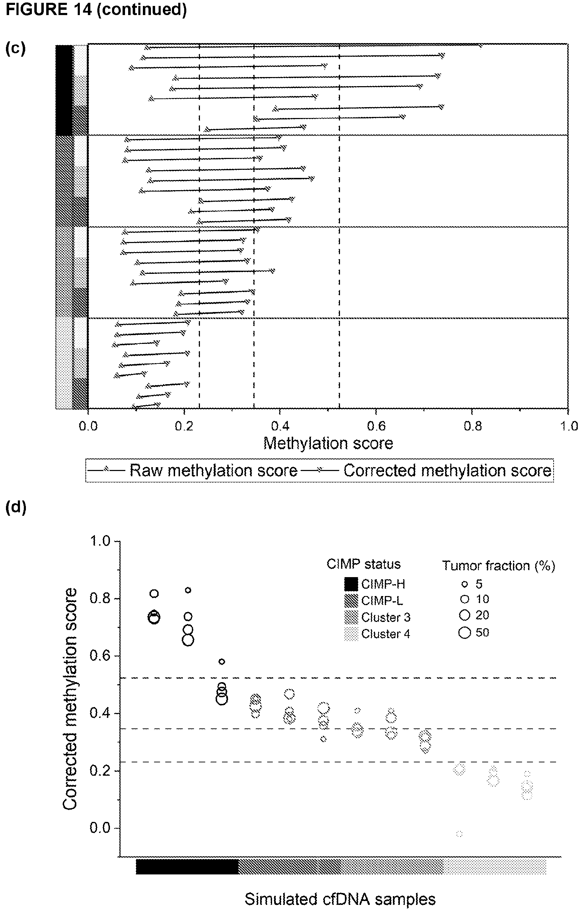

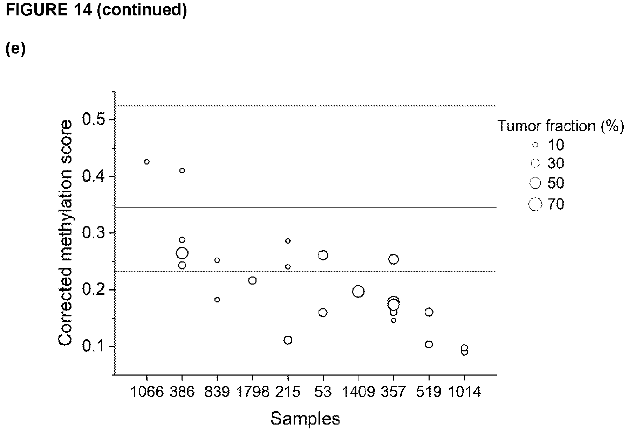

A scoring system was then developed that would allow easy classification of tumor methylation subtypes (Example 1). Applying this scoring system to the 233 TCGA CRC samples [Muzny, D. M. et al. Nature 487:330-337 (2012)], we observed that CIMP subtypes of CRC tissues are well-defined by ranges of Methylation Scores, and a series of threshold values in Methylation Scores allows 89% accuracy in classifying CIMP subtypes (Fig. 14b). Nevertheless, raw Methylation Scores from cfDNA are not expected to reflect the methylation subtype of the underlying tumor since cfDNA is derived from mixture of normal and cancerous cells. To validate the approach used to determine methylation score of the underlying tumor in cfDNA, we simulated cfDNA containing different tumor methylation subtypes at varying tumor fractions by creating mixtures of sequencing reads drawn from TCGA and healthy plasma methylation measurements (Fig. 14c). While the raw Methylation Score from cfDNA is confounded by its tumor fraction, incorporating tumor fraction estimation from Heatrich-BS assay enables the calculation of corrected Methylation Score that accurately reflected the underlying tumor methylation subtype (86% accuracy when tumor fraction is above 10%). Tumor fractions below 10% exhibited higher uncertainty in tumor methylation subtype prediction due to fewer tumor-derived cfDNA fragments (Fig. 14d). Finally, we applied this algorithm to infer tumor methylation subtype of our longitudinal tracking cohort (Fig. 14e). We calculated Methylation Score for 24 of 79 samples that had tumor fractions >10%. Our results showed that the corrected Methylation Scores of longitudinal samples from the same patient are often tightly clustered and independent of tumor fraction, while Methylation Scores between patients could differ greatly, suggesting that the methylation subtype of a patient tumor does not change significantly through disease progression. Our results predicted that there were no CIMP-High patients in the profiled cohort, with most patients falling into CIMP-negative subtypes, Cluster 3 or Cluster 4. It has been reported that CIMP-High tumors in CRC are strongly associated with microsatellite instability [Weisenberger, D. J. et al. Nat Genet 38:787-793 (2006)]. Our tumor methylation subtype prediction from cfDNA is well in line with expectation as all the patient tumors in this longitudinal cohort were profiled to be microsatellite stable during standard clinical evaluation (Table 1).

### Summary

The present invention provides the first assay that utilizes the concept of thermal denaturation to achieve CpG enrichment in fragmented DNA, which we call the Heatrich-BS assay. Heatrich selects for DNA fragments with GC content exceeding 60%, and nearly 30% of Heatrich-BS reads are in CGls, which comprise less than 1% of the genome. We also developed a tumor fraction prediction algorithm to augment our assay and validated its application for tumor fractions as low as 0.5% from low-depth sequencing (1X). With this two-pronged approach, we realized a universal low cost ($30) cfDNA methylation assay for quantitative cancer detection.

The invention provides accurate tumor fraction estimates that correspond to cfDNA mutation and copy number measurements. Due to its sensitivity and low cost, the invention is particularly amenable to non-invasive monitoring of cancer progression or recurrence, in which frequent measurements are needed and current methods are inadequate. The invention can provide superior sensitivity for detection of CRC at low tumor fractions compared to conventional CEA protein biomarker assay in longitudinal monitoring of cancer patients. In addition, we demonstrated for the first time the elucidation of tumor methylation subtypes from cfDNA, further confirming the advantage of broad genomic coverage using the invention.

The Heatrich-BS method offers significant advantages compared to current assays: (i) The workflow of Heatrich-BS is short and easy to perform. The entire assay, from sample collection to sequencing, can be performed in less than 48 hours, resulting in a short turnaround time even for a sequencing assay. (ii) Heat denaturation is independent of DNA sequence biases that can arise from the use of restriction enzymes in assays like RRBS. (iii) Heatrich is based on GC content, which is a physical property of DNA. This enables effective CpG enrichment even in fragmented DNA, such as cfDNA and FFPE samples, where the enrichment capability of current assays such as RRBS are limited [Ludgate, J. L. et al. BMC Med Genomics 10:1-10 (2017)]. (iv) Heatrich-BS requires fewer sequencing reads compared to conventional untargeted assays, which makes it highly cost-effective (>10-fold cost saving) to perform.

Finally, the extensive coverage of epigenetically informative regions using Heatrich-BS assay could enable exploration of other important applications. The vast majority (83%) of tissue-specific methylation haplotype blocks (MHBs) [Guo, S et al. Nat Genet 49:635-642 (2017)] identified in previous reports could be detected using Heatrich-BS, suggesting a potential for its use as a universal, affordable multi-cancer screening and discrimination assay. We envision that the novel Heatrich-BS platform would be an important innovation to enable practical and scalable implementation of cfDNA methylation profiling in liquid biopsy for clinical translation.

### References

Adalsteinsson, V. A. et al. Scalable whole-exome sequencing of cell-free DNA reveals high concordance with metastatic tumors. Nat. Commun. 8, (2017).
Andrews, S. FastQC: a quality control tool for high throughput sequence data. (2010).
Berthold, M. R. et al. KNIME: The Konstanz Information Miner. in Studies in Classification, Data Analysis, and Knowledge Organization. GfKL (Springer, 2007).
Bos, J.L., Fearon,E.R., Hamilton,S.R., Vries,M.V., van Boom,J.H., van der Eb,A.J. and Vogelstein,B. (1987) Prevalence of ras gene mutations in human colorectal cancers. Nature, 327, 293-297.
Broad Institute. Picard Tools. (2018). broadinstitutedotgithubdotio/picard/
Diehl, F., Li,M., He,Y., Kinzler,K.W., Vogelstein,B. and Dressman,D. (2006) BEAMing: single-molecule PCR on microparticles in water-in-oil emulsions. Nat. Methods, 3, 551-559.
Doherty, A. J. & Wigley, D. B. Functional domains of an ATP-dependent DNA ligase. J. Mol. Biol. 285, 63-71 (1999).
Forshew,T., Murtaza,M., Parkinson,C., Gale,D., Tsui,D.W.Y., Kaper,F., Dawson,S.J., Piskorz, A.M., Jimenez-Linan,M., Bentley,D., et al. (2012) Noninvasive identification and monitoring of cancer mutations by targeted deep sequencing of plasma DNA. Sci. Transl. Med., 4(136):136ra68.
Fiala, C. and Diamandis,E.P. (2018) Utility of circulating tumor DNA in cancer diagnostics with emphasis on early detection. BMC Med., 16, 1-10.
Gaspar, J. M. & Hart, R. P. DMRfinder: Efficiently identifying differentially methylated regions from MethylC-seq data. BMC Bioinformatics 18, 1-8 (2017).
Gu, H., Smith,Z.D., Bock,C., Boyle,P., Gnirke,A. and Meissner,A. (2011) Preparation of reduced representation bisulfite sequencing libraries for genome-scale DNA methylation profiling. Nat. Protoc., 6, 468-481.
Guo, H., Zhu,P., Guo,F., Li,X., Wu,X., Fan,X., Wen,L. and Tang,F. (2015) Profiling DNA methylome landscapes of mammalian cells with single-cell reduced-representation bisulfite sequencing. Nat. Protoc., 10, 645-59.
Guo, S., Diep,D., Plongthongkum,N., Fung,H.L., Zhang,K. and Zhang,K. (2017) Identification of methylation haplotype blocks AIDS in deconvolution of heterogeneous tissue samples and tumor tissue-of-origin mapping from plasma DNA. Nat. Genet., 49, 635-642.
Johnson, D. A. et al. Plasma Septin9 versus fecal immunochemical testing for colorectal cancer screening: a prospective multicenter study. PLoS One 9, e98238 (2014).
Krueger, F. & Andrews, S. R. Bismark: A flexible aligner and methylation caller for Bisulfite-Seq applications. Bioinformatics 27, 1571-1572 (2011).
Leary, R. J. et al. Integrated analysis of homozygous deletions, focal amplifications, and sequence alterations in breast and colorectal cancers. Proc. Natl. Acad. Sci. U. S. A. 105, 16224-9 (2008).
Li, W. et al. CancerDetector: ultrasensitive and non-invasive cancer detection at the resolution of individual reads using cell-free DNA methylation sequencing data. Nucleic Acids Res. 46, e89 (2018).
Liu, M.C., Oxnard,G.R., Klein,E.A., Swanton,C., Seiden,M.V., Cummings,S.R., Absalan,F., Alexander,G., Allen,B., Amini,H., et al. (2020) Sensitive and specific multi-cancer detection and localization using methylation signatures in cell-free DNA. Ann. Oncol., 31, 745-759.
Ludgate, J. L. et al. A streamlined method for analysing genome-wide DNA methylation patterns from low amounts of FFPE DNA. BMC Med. Genomics 10, 1-10 (2017).
Juo, Y. Y. et al. Prognostic value of CpG island methylator phenotype among colorectal cancer patients: A systematic review and meta-analysis. Ann. Oncol. 25, 2314-2327 (2014).
Mojarad, E. N., Kuppen, P. J. K., Aghdaei, H. A. & Zali, M. R. The CpG island methylator phenotype (CIMP) in colorectal cancer. Gastroenterol. Hepatol. from Bed to Bench 6, 120-128 (2013).
Moss, J., Magenheim,J., Neiman,D., Zemmour,H., Loyfer,N., Korach,A., Samet,Y., Maoz,M., Druid,H., Arner,P., et al. (2018) Comprehensive human cell-type methylation atlas reveals origins of circulating cell-free DNA in health and disease. Nat. Commun., 9(1):5068.
Mouliere, F., Chandrananda,D., Piskorz,A.M., Moore,E.K., Morris,J., Ahlborn,L.B., Mair,R., Goranova,T., Marass,F., Heider,K., et al. (2018) Enhanced detection of circulating tumor DNA by fragment size analysis. Sci. Transl. Med., 10, 1-14.
Muzny, D. M. et al. Comprehensive molecular characterization of human colon and rectal cancer. Nature 487, 330-337 (2012).
Sendorek, D. H. et al. Germline contamination and leakage in whole genome somatic single nucleotide variant detection. BMC Bioinformatics 19, 28 (2018).
Shinkins, B. et al. The diagnostic accuracy of a single CEA blood test in detecting colorectal cancer recurrence: Results from the FACS trial. PLoS One 12, (2017).
Shu, Y., Wu,X., Tong,X., Wang,X., Chang,Z., Mao,Y., Chen,X., Sun,J., Wang,Z., Hong,Z., et al. (2017) Circulating Tumor DNA Mutation Profiling by Targeted Next Generation Sequencing Provides Guidance for Personalized Treatments in Multiple Cancer Types. Sci. Rep., 7, 1-11.
Sougnez, C., Gabriel, S., Meyerson, M. & Lander, E. S. MuTect. Nat Biotechnol 31, 213-219 (2013).
Sproul, D. and Meehan,R.R. (2013) Genomic insights into cancer-associated aberrant CpG island hypermethylation. Brief. Funct. Genomics, 12, 174-190.
Sun, K. et al. Plasma DNA tissue mapping by genome-wide methylation sequencing for noninvasive prenatal, cancer, and transplantation assessments. Proc. Natl. Acad. Sci. U. S. A. 112, E5503-E5512 (2015).
Weisenberger, D. J. et al. CpG island methylator phenotype underlies sporadic microsatellite instability and is tightly associated with BRAF mutation in colorectal cancer. Nat. Genet. 38, 787-793 (2006).
Zhang, X., Zhang, W. & Cao, P. Advances in CpG Island Methylator Phenotype Colorectal Cancer Therapies. Front. Oncol. 11, (2021).

## Claims

1. A method of enrichment of CpG islands comprising cancer-specific methylation information in isolated circulating cell-free DNA from a subject, comprising the steps:
i) provide a cell-free DNA sample that has been isolated from the subject;
ii) repair double-stranded DNA ends and add dA tail;
iii) heat-denature the cell-free DNA, wherein low GC content fragments are denatured while high GC content fragments remain double-stranded;
iv) ligate methylated adapters to both ends of the double-stranded DNA;
v) perform bisulfite conversion of the adapter-ligated DNA;
vi) amplify the bisulfite-converted adapter-ligated DNA from v);
vii) Size-select for 190-400 bp fragments of the amplified DNA of vi).

2. The method of claim 1, wherein low GC content fragments have lower than about 60% GC content and high GC content fragments have about 60% or higher GC content; and/or
wherein the heat denaturing in step iii) is performed at a temperature in the range of about 87-92 °C; and/or
wherein the cell-free DNA sample is from a subject that has a medical condition; and/or
further comprising determining at least part of the sequence of one or more of the amplified molecules.

3. The method of claim 2, wherein the determining of at least part of the sequence comprises paired-end sequencing.

4. The method of claim 3, wherein the determining step provides diagnostic information for the subject.

5. The method of claim 4, wherein the diagnostic information comprises cancer diagnosis information for the subject.

6. A method of obtaining information in relation to a medical condition of a subject, the method comprising:
i) provide an isolated cell-free DNA sample from the subject;
ii) repair double-stranded DNA ends and add dA tail;
iii) heat-denature the cell-free DNA, wherein low GC content fragments are denatured while high GC content fragments remain double-stranded;
iv) ligate methylated adapters to both ends of the double-stranded DNA;
v) perform bisulfite conversion of the adapter-ligated DNA;
vi) amplify the bisulfite-converted adapter-ligated DNA from v);
vii) Size-select for 190-400 bp fragments of the amplified DNA of vi);
viii) determine at least part of the sequence of one or more of the amplified molecules;
determine the tumor fraction in the sample from the subject, comprising:
A) Identify differentially methylated clusters from a comparison of Whole Genome Bisulfite Sequencing datasets for normal plasma with a reference cancer methylation dataset;
B) Compare each CpG site in each bisulfite-sequenced heat-enriched CpG-rich DNA fragment from said subject sample against the reference dataset and calculate, using a bimodal distribution, a class-specific probability for each bisulfite-sequenced heat-enriched CpG-rich DNA fragment; and
C) Approximate the tumor fraction in said subject sample using maximum likelihood estimation.

7. The method of claim 6, wherein low GC content fragments have lower than about 60% GC content and high GC content fragments have about 60% or higher GC content.

8. The method of claim 6 or 7, comprising:
A) identifying differentially methylated clusters in circulating cell-free DNA between normal subjects and subjects with cancer by;
(i) obtaining a normal plasma whole genome bisulfite sequencing methylation dataset;
(ii) obtaining a reference cancer methylation dataset and extrapolating, to ± 100 bp of each of a plurality of probe sites, methylation values with a standard deviation less than 0.4 between the various samples in the dataset;
(iii) identifying differentially methylated clusters from datasets (i) and (ii);
B) determining the class-specific probability of each site using a bimodal distribution by;
i) assigning to each sequenced fragment from the subject sample a normal and tumor class-specific proportional methylation status using the generated reference;
ii) **for** every site in the reference, the contribution from the unmethylated and methylated mode (0 and 1) is calculated, wherein the relative contributions of each mode in the two classes is used to assign normal or tumor class-specific probabilities for the methylation values in the assayed fragment; and
C) estimating the tumor fraction, denoted as *θ*, where 0 ≤ *θ* < 1, of the sample;
wherein, each read is assumed to be independent, and normal and tumor class-specific probability is assigned to each read;
a global tumor fraction (*θ_{g}*) is calculated by applying a grid search to determine the highest probability event from a range of tumor fractions.

9. The method of claim 8, wherein in A)(iii) all CpG sites within clusters that have 0.5 difference in methylation are selected; and/or
wherein the heat denaturing in step iii) is performed at a temperature in the range of about 87-92 °C; and/or
wherein the determining of at least part of the sequence comprises paired-end sequencing; and/or
wherein the determining step provides diagnostic information for the subject.

10. The method of claim 9, wherein the diagnostic information comprises cancer diagnosis information for the subject.

11. The method of any one of claims 6 to 10, wherein the comparison population is selected from a group comprising circulating cell-free DNA from normal patients and/or cancer patients; and/or
wherein the reference cancer methylation dataset is a colorectal adenocarcinoma (COAD) dataset.

## Patentansprüche

1. Verfahren zur Anreicherung von CpG-Inseln, die krebsspezifische Methylierungsinformationen umfassen, in isolierter zirkulierender zellfreier DNA aus einem Probanden, umfassend die folgenden Schritte:
i) Bereitstellen einer zellfreien DNA-Probe, die aus dem Probanden isoliert wurde;
ii) Reparieren der Doppelstrang-DNA-Enden und Hinzufügen eines dA-Überhangs;
iii) Wärmedenaturieren der zellfreien DNA, wobei Fragmente mit niedrigem GC-Gehalt denaturiert werden, während Fragmente mit hohem GC-Gehalt doppelsträngig bleiben;
iv) Ligieren methylierter Adapter an beide Enden der doppelsträngigen DNA;
v) Durchführen einer Bisulfit-Konversion der Adapter-ligierten DNA;
vi) Amplifizieren der Bisulfit-konvertierten, Adapter-ligierten DNA aus v);
vii) größenspezifisches Auswählen von 190-400 bp großen Fragmenten der amplifizierten DNA aus vi).

2. Verfahren nach Anspruch 1, wobei Fragmente mit niedrigem GC-Gehalt einen GC-Gehalt von weniger als etwa 60 % und Fragmente mit hohem GC-Gehalt einen GC-Gehalt von etwa 60 % oder mehr aufweisen; und/oder
wobei die Wärmedenaturierung in Schritt iii) bei einer Temperatur im Bereich von etwa 87-92 °C durchgeführt wird; und/oder
wobei die zellfreie DNA-Probe von einem Probanden stammt, der einen Gesundheitszustand aufweist; und/oder
ferner umfassend das Bestimmen mindestens eines Teils der Sequenz eines oder mehrerer der amplifizierten Moleküle.

3. Verfahren nach Anspruch 2, wobei das Bestimmen mindestens eines Teils der Sequenz eine Paired-End-Sequenzierung umfasst.

4. Verfahren nach Anspruch 3, wobei der Schritt des Bestimmens diagnostische Informationen über den Probanden bereitstellt.

5. Verfahren nach Anspruch 4, wobei die diagnostischen Informationen Krebsdiagnoseinformationen für den Probanden umfassen.

6. Verfahren zum Erhalten von Informationen über einen Gesundheitszustand eines Probanden, wobei das Verfahren Folgendes umfasst:
i) Bereitstellen einer isolierten zellfreien DNA-Probe aus dem Probanden;
ii) Reparieren der Doppelstrang-DNA-Enden und Hinzufügen eines dA-Überhangs;
iii) Wärmedenaturieren der zellfreien DNA, wobei Fragmente mit niedrigem GC-Gehalt denaturiert werden, während Fragmente mit hohem GC-Gehalt doppelsträngig bleiben;
iv) Ligieren von methylierten Adaptern an beide Enden der doppelsträngigen DNA;
v) Durchführen einer Bisulfit-Konversion der Adapter-ligierten DNA;
vi) Amplifizieren der Bisulfit-konvertierten, Adapter-ligierten DNA aus v);
vii) Auswählen der Größe von 190-400 bp großen Fragmenten der amplifizierten DNA aus vi);
viii) Bestimmen mindestens eines Teils der Sequenz eines oder mehrerer der amplifizierten Moleküle;
Bestimmen der Tumorfraktion in der Probe des Probanden, umfassend:
A) Identifizieren differentiell methylierter Cluster durch einen Vergleich von Whole Genome-Bisulfit-Sequenzierung-Datensätzen für normales Plasma mit einem Referenz-Krebsmethylierung-Datensatz;
B) Vergleichen jeder CpG-Stelle in jedem bisulfitsequenzierten, wärmeangereicherten, CpG-reichen DNA-Fragment der Probe des Probanden mit dem Referenz-Datensatz und Berechnen, unter Verwendung einer bimodalen Verteilung, einer klassenspezifischen Wahrscheinlichkeit für jedes bisulfitsequenzierte, wärmeangereicherte, CpG-reiche DNA-Fragment; und
C) Approximieren der Tumorfraktion in der Probe des Probanden mittels Maximum-Likelihood-Schätzung.

7. Verfahren nach Anspruch 6, wobei Fragmente mit niedrigem GC-Gehalt einen GC-Gehalt von weniger als etwa 60 % aufweisen und Fragmente mit hohem GC-Gehalt einen GC-Gehalt von etwa 60 % oder mehr aufweisen.

8. Verfahren nach Anspruch 6 oder 7, umfassend:
A) Identifizieren differentiell methylierter Cluster in zirkulierender zellfreier DNA zwischen normalen Probanden und Probanden mit Krebs durch;
(i) Erhalten eines Whole Genome-Bisulfit-Sequenzierung-Methylierung-Datensatzes für normales Plasma;
(ii) Erhalten eines Referenzkrebs-Methylierung-Datensatzes und Extrapolieren der Methylierungswerte auf ± 100 bp jeder der Vielzahl von Sondenstellen mit einer Standardabweichung von weniger als 0,4 zwischen den verschiedenen Proben im Datensatz;
(iii) Identifizieren differentiell methylierter Cluster aus den Datensätzen (i) und (ii);
B) Bestimmen der klassenspezifischen Wahrscheinlichkeit jeder Stelle unter Verwendung einer bimodalen Verteilung durch;
i) Zuordnen, zu jedem sequenzierten Fragment aus der Probe des Probanden, eines normalen und tumorklassenspezifischen proportionalen Methylierungsstatus unter Verwendung der generierten Referenz;
ii) wobei, für jede Stelle in der Referenz der Beitrag des unmethylierten und des methylierten Modus (0 und 1) berechnet wird, wobei die relativen Beiträge jedes Modus in den beiden Klassen verwendet werden, um normale oder tumorklassenspezifische Wahrscheinlichkeiten für die Methylierungswerte im untersuchten Fragment zuzuordnen; und
C) Schätzen der Tumorfraktion, bezeichnet als *θ*, wobei 0 ≤ *θ* < 1 der Probe;
wobei davon ausgegangen wird, dass jeder Messwert unabhängig ist und jedem Messwert eine normale und tumorklassenspezifische Wahrscheinlichkeit zugeordnet wird;
eine globale Tumorfraktion (*θ_{g}*) berechnet wird, indem eine Gittersuche angewendet wird, um das Ereignis mit der höchsten Wahrscheinlichkeit aus einer Reihe von Tumorfraktionen zu bestimmen.

9. Verfahren nach Anspruch 8, wobei in A) (iii) alle CpG-Stellen innerhalb von Clustern ausgewählt werden, die einen Methylierungsunterschied von 0,5 aufweisen; und/oder
wobei die Wärmedenaturierung in Schritt iii) bei einer Temperatur im Bereich von etwa 87 bis 92 °C durchgeführt wird; und/oder
wobei die Bestimmung mindestens eines Teils der Sequenz eine Paired-End-Sequenzierung umfasst; und/oder
wobei der Schritt des Bestimmens diagnostische Informationen für den Probanden bereitstellt.

10. Verfahren nach Anspruch 9, wobei die diagnostischen Informationen Krebsdiagnoseinformationen für den Probanden umfassen.

11. Verfahren nach einem der Ansprüche 6 bis 10, wobei die Vergleichspopulation aus einer Gruppe ausgewählt wird, die zirkulierende zellfreie DNA von normalen Patienten und/oder Krebspatienten umfasst; und/oder
wobei es sich bei dem Referenz-Krebsmethylierung-Datensatz um einen kolorektalen Adenokarzinom-Datensatz (COAD-Datensatz) handelt.

## Revendications

1. Procédé d'enrichissement d'îlots CpG comprenant des informations de méthylation spécifique au cancer dans de l'ADN acellulaire circulant isolé provenant d'un sujet, comprenant les étapes consistant à :
i) la fourniture d'un échantillon d'ADN acellulaire qui a été isolé du sujet ;
ii) la réparation d'extrémités de l'ADN double brin et ajouter une queue dA ;
iii) la dénaturation thermique de l'ADN acellulaire, dans lequel des fragments à faible teneur en GC sont dénaturés tandis que des fragments à teneur élevée en GC restent double brin ;
iv) la ligature d'adaptateurs méthylés aux deux extrémités de l'ADN double brin ;
v) la réalisation d'une conversion au bisulfite de l'ADN ligaturé à l'adaptateur ;
vi) l'amplification de l'ADN ligaturé à l'adaptateur converti au bisulfite provenant de v) ;
vii) la sélection d'une taille pour des fragments de 190 à 400 pb de l'ADN amplifié de vi).

2. Procédé selon la revendication 1, dans lequel des fragments à faible teneur en GC présentent une teneur en GC inférieure à environ 60 % et des fragments à teneur élevée en GC présentent une teneur en GC d'environ 60 % ou plus ; et/ou
dans lequel la dénaturation thermique à l'étape iii) est réalisée à une température dans la plage d'environ 87 à 92 °C ; et/ou
dans lequel l'échantillon d'ADN acellulaire provient d'un sujet qui présente une affection médicale ; et/ou
comprenant en outre la détermination d'au moins une partie de la séquence d'une ou plusieurs des molécules amplifiées.

3. Procédé selon la revendication 2, dans lequel la détermination d'au moins une partie de la séquence comprend un séquençage à extrémités appariées.

4. Procédé selon la revendication 3, dans lequel l'étape de détermination fournit des informations de diagnostic pour le sujet.

5. Procédé selon la revendication 4, dans lequel les informations de diagnostic comprennent des informations de diagnostic de cancer pour le sujet.

6. Procédé d'obtention d'informations relatives à un état de santé d'un sujet, le procédé comprenant :
i) la fourniture d'un échantillon d'ADN acellulaire isolé provenant du sujet ;
ii) la réparation d'extrémités d'ADN double brin et l'ajout d'une queue dA ;
iii) la dénaturation thermique de l'ADN acellulaire, dans lequel des fragments à faible teneur en GC sont dénaturés tandis que des fragments à teneur élevée en GC restent double brin ;
iv) la ligature d'adaptateurs méthylés aux deux extrémités de l'ADN double brin ;
v) la réalisation d'une conversion au bisulfite de l'ADN ligaturé à l'adaptateur ;
vi) l'amplification de l'ADN ligaturé à l'adaptateur converti au bisulfite provenant de v) ;
vii) la sélection d'une taille pour des fragments de 190 à 400 pb de l'ADN amplifié de vi) ;
viii) la détermination d'au moins une partie de la séquence d'une ou plusieurs des molécules amplifiées ;
la détermination de la fraction tumorale dans l'échantillon provenant du sujet comprenant :
A) l'identification de clusters méthylés de manière différentielle à partir d'une comparaison d'ensembles de données de séquençage au bisulfite du génome entier pour du plasma normal avec un ensemble de données de référence sur la méthylation du cancer ;
B) la comparaison de chaque site CpG dans chaque fragment d'ADN riche en CpG enrichi par la chaleur et séquencé au bisulfite provenant dudit échantillon de sujet par rapport à l'ensemble de données de référence et le calcul, à l'aide d'une distribution bimodale, d'une probabilité spécifique à la classe pour chaque fragment d'ADN riche en CpG enrichi par la chaleur et séquencé au bisulfite ; et
C) l'approximation de la fraction tumorale dans ledit échantillon de sujet en utilisant une estimation du maximum de vraisemblance.

7. Procédé selon la revendication 6, dans lequel des fragments à faible teneur en GC présentent une teneur en GC inférieure à environ 60 % et des fragments à teneur élevée en GC présentent une teneur en GC d'environ 60 % ou plus.

8. Procédé selon la revendication 6 ou la revendication 7, comprenant :
A) l'identification de clusters méthylés de manière différentielle dans de l'ADN acellulaire circulant entre des sujets normaux et des sujets atteints de cancer par ;
(i) l'obtention d'un ensemble de données de méthylation par séquençage au bisulfite du génome entier du plasma normal ;
(ii) l'obtention d'un ensemble de données de référence sur la méthylation du cancer et extrapolation, à ± 100 pb de chacun d'une pluralité de sites de sonde, de valeurs de méthylation avec un écart type inférieur à 0,4 entre les différents échantillons dans l'ensemble de données ;
(iii) l'identification de clusters méthylés de manière différentielle à partir des ensembles de données (i) et (ii) ;
B) la détermination de la probabilité spécifique à la classe de chaque site en utilisant une distribution bimodale par ;
i) l'attribution à chaque fragment séquencé provenant de l'échantillon de sujet d'un statut de méthylation proportionnel normal et spécifique à la classe tumorale en utilisant la référence générée ;
ii ) pour chaque site dans la référence, la contribution du mode non méthylé et du mode méthylé (0 et 1) est calculée, dans lequel les contributions relatives de chaque mode dans les deux classes sont utilisées pour attribuer des probabilités spécifiques à la classe normale ou tumorale pour les valeurs de méthylation dans le fragment analysé ; et
C) l'estimation de la fraction tumorale, notée *θ*, où 0 ≤ *θ* < 1, de l'échantillon ;
dans lequel chaque lecture est supposée indépendante et une probabilité spécifique à la classe normale et tumorale est attribuée à chaque lecture ;
une fraction tumorale globale (*θ_{g}*) est calculée en appliquant une recherche par grille pour déterminer l'événement le plus probable parmi une plage de fractions tumorales.

9. Procédé selon la revendication 8, dans lequel dans A)(iii) tous les sites CpG au sein de clusters qui présentent une différence de méthylation de 0,5 sont sélectionnés ; et/ou
dans lequel la dénaturation thermique à l'étape iii) est réalisée à une température dans la plage d'environ 87 à 92 °C ; et/ou
dans lequel la détermination d'au moins une partie de la séquence comprend un séquençage à extrémités appariées ; et/ou
dans lequel l'étape de détermination fournit des informations de diagnostic pour le sujet.

10. Procédé selon la revendication 9, dans lequel les informations de diagnostic comprennent des informations de diagnostic de cancer pour le sujet.

11. Procédé selon l'une quelconque des revendications 6 à 10, dans lequel la population de comparaison est sélectionnée parmi un groupe comprenant de l'ADN acellulaire circulant provenant de patients normaux et/ou de patients atteints de cancer ; et/ou
dans lequel l'ensemble de données de référence sur la méthylation du cancer est un ensemble de données sur l'adénocarcinome colorectal (COAD).
